# EUROPEAN PATENT SPECIFICATION

(11) **EP 1 513 831 B1**
(45) Date of publication and mention of the grant of the patent: **06.01.2010**
(21) Application number: 03760997.1
(22) Date of filing: 18.06.2003
(51) Int. Cl.: C07D 401/14, C07D 401/12, C07D 401/04, C07D 401/08, C07D 403/04, C07D 403/14, C07D 241/12, C07D 241/20, A61K 31/444, A61K 31/497, A61P 25/00

(54) **NOVEL COMPOUNDS, THEIR USE AND PREPARATION**
NEUE VERBINDUNGEN, DEREN VERWENDUNG UND HERSTELLUNG
NOUVEAUX COMPOSES, LEUR UTILISATION ET LEUR PREPARATION

(30) Priority: 19.06.2002 SE 0201881; 26.08.2002 SE 0202516; 26.08.2002 US 406119 P; 07.10.2002 US 416701 P
(43) Date of publication of application: 16.03.2005
(73) Proprietor: Biovitrum AB (publ), 112 76 Stockholm (SE)
(72) Inventor: NILSSON, Björn, S-112 49 Stockholm (SE); RINGBERG, Erik, S-757 55 Uppsala (SE); JÖNSSON, Mattias, S-756 49 Uppsala (SE); SJÖBERG, Birger, S-191 46 Sollentuna (SE)
(74) Representative: Höglund, Lars
(86) International application number: PCT/SE2003/001044
(87) International publication number: WO 2004/000830

(56) References cited:
- WO-A1-02/40456
- WO-A1-02/40457
- WO-A1-03/000663
- WO-A2-00/76984
- LUMMA WILLIAM C. ET AL.: 'Piperazinylpyrazines with central serotoninmimetic activity' JOURNAL OF MEDICINAL CHEMISTRY vol. 21, no. 6, 1978, pages 536 - 542, XP002933691

## Description

### Field of the invention

The present invention relates to novel compounds, to pharmaceutical compositions comprising the compounds, to processes for their preparation, as well as to the use of the compounds for the preparation of a medicament which particularly acts on the central nervous system.

### Background of the invention

Many disorders and conditions of the central nervous system are influenced by the adrenergic, the dopaminergic, and the serotonergic neurotransmitter systems. For example, serotonin has been implicated in a number of disorders and conditions which originate in the central nervous system. A number of pharmacological and genetic experiments involving receptors for serotonin strongly implicate the 5-HT_{2C} receptor subtype in the regulation of food intake (see e. g., Obes. Res. 1995, 3, Suppl. 4, 449S-462S,Diabetes, Obesity and Metabolism 1999, 1,207-214, and Drugs Future 2001, 26, 383-393). The 5-HT_{2C} receptor subtype is transcribed and expressed in hypothalamic structures associated with appetite regulation. It has been demonstrated that the 5-HT_{2C} receptor agonist m-chlorophenyl-piperazine (mCPP), which has some preference for the 5-HT_{2C} receptor, reduces food intake in mice that express the normal 5-HT_{2C} receptor while the compound lacks activity in mice expressing the mutated inactive form of the 5-HT_{2C} receptor (Nature 1995, 374, 542-546). In a recent clinical study, a slight but sustained reduction in body weight was obtained after 2 weeks of treatment with mCPP in obese subjects (Psychopharmacology 1997, 133, 309-312). Recently, a series of pyrrolo[3,2,1-ij]quinoline derivatives was identified to be 5-HT_{2C} receptor agonists having selectivity over the 5-HT_{2A} receptor (Isaac M., et al., Bioorg. Med. Chem. Lett. 2000, 10, 919-921). The compounds are said to offer a novel approach to the treatment of obesity and epilepsy.

Body weight reduction has also been reported from clinical studies with other "serotonergic" agents (see e.g. IDrugs 1998, 1, 456-470). For example, the 5-HT reuptake inhibitor fluoxetine and the 5-HT releasing agent/reuptake inhibitor dexfenfluramine have exhibited weight reduction in controlled studies. However, currently available drugs that increase serotonergic transmission appear to have only a moderate and, in some cases, transient effects on the body weight.

The 5-HT_{2C} receptor subtype has also been suggested to be involved in CNS disorders such as depression and anxiety (Exp. Opin. Invest. Drugs 1998, 7, 1587-1599; IDrugs, 1999, 2, 109-120).

The 5-HT_{2C} receptor subtype has further been suggested to be involved in urinary disorders such as urinary incontinence (IDrugs, 1999, 2, 109-120).

Compounds which have a selective effect on the 5-HT_{2C} receptor may therefore have a therapeutic potential in the treatment of disorders like those mentioned above. Of course, selectivity also reduces the potential for adverse effects mediated by other serotonin receptors.

### Information disclosure

US-A-3,253,989 discloses the use of mCPP as an anorectic agent.

EP-A1-863 136 discloses azetidine and pyrrolidine derivatives which are selective 5-HT_{2C} receptor agonists having antidepressant activity and which can be used for treating or preventing serotonin-related diseases, including eating disorders and anxiety.

EP-A-657 426 discloses tricyclic pyrrole derivatives having activity on the 5-HT_{2C} receptor and which inter alia may be used for treating eating disorders.

EP-A-655 440 discloses 1-aminoethylindoles having activity on the 5-HT_{2C} receptor and which may be used for treating eating disorders.

EP-A-572 863 discloses pyrazinoindoles having activity on the 5-HT_{2C} receptor and which may be used for treating eating disorders.

J. Med. Chem. 1978, 21,536-542 and US-A-4,081,542 disclose a series of piperazinylpyrazines having central serotonin-mimetic activity. In particular, US-A-4,081,542 discloses such compounds as anorectic agents.

J. Med. Chem. 1981, 24, 93-101 discloses a series of piperazinylquinoxalines with central serotonin-mimetic activity.

WO 00/12475 discloses indoline derivatives as 5-HT_{2B} and/or 5-HT_{2C} receptor ligands, especially for the treatment of obesity.

WO 00/12510 discloses pyrroloindoles, pyridoindoles and azepinoindoles as 5-HT_{2C} receptor agonists, particularly for the treatment of obesity.

WO 00/12482 discloses indazole derivatives as selective, directly active 5-HT_{2C} receptor ligands, preferably 5-HT_{2C} receptor agonists, particularly for use as anti-obesity agents.

WO 00/12502 discloses pyrroloquinolines as 5-HT_{2C} receptor agonists, particularly for use as anti-obesity agents.

GB-B-1,457,005 discloses 1-piperazinyl-2-[2-(phenyl)ethenyl]-quinoxaline derivatives which exhibit anti-inflammatory activity.

Chem. Pharm. Bull. 1993, 41(10) 1832-1841 discloses 5-HT₃ antagonists including 2-(4-methyl-1-piperazinyl)-4-phenoxyquinoxaline.

GB-B-1,440,722 discloses 2-(1'-piperazinyl)-quinoxaline compounds having pharmaceutical activity against depression.

WO 96/11920 discloses CNS-active pyridinylurea derivatives.

WO 95/01976 discloses indoline derivatives active as 5-HT_{2C} antagonists and of potential use in the treatment of CNS disorders.

WO 97/14689 discloses aryl-piperazine cyclic amine derivatives which are selective 5-HT_{1D} receptor antagonists.

WO 98/42692 discloses piperazines derived from cyclic amines which are selective antagonists of human 5-HT_{1A}, 5-HT_{1D} and 5-HT_{1B} receptors.

GB-B-1,465,946 discloses substituted pyridazinyl, pyrimidinyl, pyrazinyl and pyridyl compounds which are active as β-receptor blocking agents.

EP-A-711757 discloses [3-(4-phenyl-piperazin-1-yl)propylamino]-pyridine, pyrimidine and benzene derivatives as α-adrenoceptor antagonists.

WO 99/03833 discloses aryl-piperazine derivatives which are 5-HT₂ antagonists and 5-HT_{1A} receptor agonists and therefore are useful as remedies or preventives for psychoneurosis.

WO 96/02525 discloses aryl-piperazine-derived piperazide derivatives having 5-HT receptor antagonistic activity.

WO 99/58490 disloses aryl-hydronaphthalen-alkanamines which may effectuate partial or complete blockage of serotonergic 5-HT_{2C} receptors in an organism.

WO 00/35922 discloses 2,3,4,4a-tetrahydro-1*H*-pyrazino[1,2-*a*]quinoxalin-5(6*H*)ones as 5-HT_{2C} agonists, which may be used for the treatment of obesity.

WO 00/44737 discloses aminoalkylbenzofurans as 5-HT_{2C} agonists, which may be used for the treatment of obesity.

Further compounds reported to be 5-HT_{2C} receptor agonists are, for example, indazolylpropylamines of the type described in WO 00/12481; indazoles of the type described in WO 00/17170; piperazinylpyrazines of the type described in WO 00/76984, WO 02/40456 and WO 02/40457; heterocycle fused γ-carbolines of the type described in WO 00/77001, WO 00/77002 and WO 00/77010; benzofurylpiperazines of the type described in WO 01/09111 and WO 01/09123; benzofurans of the type described in WO 01/09122; benzothiophenes of the type described in 01/09126; aminoalkylindazoles of the type described in WO 98/30548; indoles of the type described in WO 01/12603; indolines of the type described in WO 01/12602 and WO 02/44152; pyrazino(aza)indoles of the type described in WO 00/44753; diaza-cyclopenta[a]indenes of the type described in EP 1132389; piperazine derivatives of the type described in WO 02/10169; quinoxalinones of the type described in US 6372745, and tricyclic pyrroles or pyrazoles of the type described in WO 98/56768.

WO 98/33504 discloses a new medical use of 1-[6-chloro-5-(trifluoromethyl)-2-pyridinyl]piperazine, in particular to a new method of treating urinary incontinence.

WO 02/36596 discloses cycloalkyl[b][1,4]-diazepino[6,7-hi]indoles as serotonin 5-HT_{2C} receptor agonists, which may be used for the treatment of obesity.

WO 03/00666 discloses [1,2']bipyrazinyl 5-HT₂ receptor ligands, in particular 5-HT_{2c} receptor ligands, for treatment of sexual dysfunction.

WO 03/00663 discloses piperazinylpyrimidines as 5-HT₂ receptor ligands, in particular 5-HT_{2c} receptor ligands, for treatment of sexual disorders.

WO 02/51844 discloses cycloalkyl fused indole derivatives and their use as 5-HT_{2b} and 5-HT_{2c} receptor ligands.

WO 02/42304 discloses cyclopenta[b][1,4]diazepino[6,7,1-hi]indoles as selective 5-HT_{2c} receptor agonists.

WO 02/36596 discloses diazepinocarbazoles and related compounds as serotonin 5-HT_{2c} agonists.

WO 02/48124 discloses piperazine derivatives as 5-HT_{2c} receptor agonists; which may be used for, e.g., obesity.

WO 01/66548 discloses azaindolyl derivatives as 5-HT_{2b} and 5-HT_{2c} receptor ligands, preferably 5-HT_{2c} receptor agonists, for use in therapy, especially for use as anti-obesity agents.

WO 02/072584 discloses tetrahydropyrazinoindoles as 5-HT_{2b} and 5-HT_{2c} receptor ligands, preferably 5-HT_{2c} receptor agonists, for use in therapy, especially for use as anti-obesity agents.

WO 00/76984 and WO 02/40457 disclose aryl-piperazine derivatives which bind to the 5-HT_{2C} receptor (agonists and antagonists) useful for the treatment of serotonin-related disorders. However, the 5-HT_{2C} receptor selectivity of the compounds according to the present invention is unexpectedly high compared to the compounds according to WO 00/76984 and WO 02/40457.

The absence of appreciable affinity to certain other 5-HT receptor subtypes may provide the basis for an improved therapeutic index compared with the general activation of all the different 5-HT receptor subtypes by 5-HT reuptake inhibitors and/or 5-HT releasing agents. The anti-obesity drug dexfenfluramine, a 5-HT reuptake inhibitor and 5-HT releaser, was withdrawn from the market in September 1997 because of several reports suggesting the association of this drug with a risk of primary pulmonary hypertension and of heart valve abnormalities (see, e.g. Kolanowski, J. A risk-benefit assessment of anti-obesity drugs. Drug Safety 1999, 20, 119-131). The 5-HT/noradrenaline reuptake inhibitor sibutramine, currently on the market for the treatment of obesity, can increase blood pressure and heart rate in some patients. The use of sibutramine has been suspended in Italy in March 2002 because of two cardiovascular deaths and its safety is currently under review in other European countries where, in the UK and France alone, there have been a total of 103 serious adverse reaction reports in people using the drug including two deaths in Britain. Taken together, there is a need for the development of safer anti-obesity agents.

It is important to minimize potential adverse advents due to activation of 5-HT_{2A} and 5-HT_{2B} receptor subtypes. 5-HT_{2A} receptor agonism is associated with vasoconstriction, platelet aggregation and hallucinogenic episodes and 5-HT_{2B} receptor agonism might play a role in the pathophysiology of migraine. Stimulation of 5-HT_{2A} and 5-HT_{2B} receptors might also have a link to cardiac fibrosis.

The following references show that hallucinogenic effects are associated with activation of the 5-HT_{2A} receptor:
(a) Glennon, R.A. et al. Hallucinogens and Serotonergic Mechanisms. NIDA Res. Mongr. 1992, 119P, 131-135.
(b) Egan, C.T. et al. Agonist Activity of LSD and Lisuride at cloned 5-HT2A and 5-HT2C receptors. Psychopharmacol. 1998, 136, 409-414.
(c) Roth, B.L. et al. The multiplicity of serotonin receptors: Uselessly diverse molecules or an embarrassment of riches? Neuroscientist 2000, 6, 252-262.
(d) Arvanov, V.L. et al. LSD and DOB: interaction with 5-HT2A receptors to inhibit NMDA receptor-mediated transmission in the rat prefrontal cortex. Eur. J. Neurosci. 1999, 11, 3064-3072.
(e) Marek, G.J. et al. LSD and the phenethylamine hallucinogen DOI are potent partial agonists at 5-HT2A receptors on interneurons in rat piriform cortex. J. Pharmacol. Exp. Ther. 1996, 278, 1373-1382.
(f) Roth, B.L. et al. Activation is hallucinogenic and antagonism is therapeutic: role of 5-HT2A receptors in atypical antipsychotic drug actions. Neuroscientist 1999, 5, 254-262.
(g) Aghajanian, G.K. et al. Serotonin and Hallucinogens. Neuropsychopharmacology 1999, 21, 16S-23S.
(h) Aghajanian, G.K. et al. Serotonin model of schizophrenia: emerging role of glutamate mechanisms. Brain Res. Rev. 2000, 31, 302-312.

The following references show that vasoconstrictive effects are associated with activation of the 5-HT_{2A} receptor:
(a) Roth, B.L. et al. 5-HT2-family receptors (5-HT2A, 5-HT2B, 5-HT2C): where structure meets function. Pharmacol. Ther. 1998, 79, 231-257.
(b) Florian, J.A. et al. Integration of mitogen-activated protein kinase activation in vascular 5-hydroxytryptamine 2A receptor signal transduction. J. Pharmacol. Exp. Ther. 1998, 284, 346-355.
(c) Saxena, P.R. Serotonin receptors: Subtypes, functional responses and therapeutic relevance. Pharmacol. Ther. 1995, 66, 339-368.
(d) MacLennan, S.J. 5-HT receptors in the human cardiovascular system. ID Res. Alert 1997, 2,207-213.
(e) Nilsson, T. et al. Characterisation of 5-HT receptors in human coronary arteries by molecular and pharmacological techniques. Eur. J. Pharmacol. 1999, 372, 49-56.
(f) MacLean, M.R. et al. 5-Hydroxytryptamine receptors mediating vasoconstriction in pulmonary arteries from control and pulmonary hypertensive rats. Br. J. Pharmacol. 1996, 119, 917-930.
(g) Cortijo, J. et al. Characterization of 5-HT receptors on human pulmonary artery and vein: functional and binding studies. Br. J. Pharmacol. 1997, 122, 1455-1463.
(h) O'Connor, S.E. et al. Cardiovascular effects of SL65.0472, a 5-HT receptor antagonist. Eur. J. Pharmacol. 2001, 414, 259-269.
(i) Galzin, A.-M. et al. Effects of SL 65.0472, a novel 5-HT receptor antagonist, on 5-HT receptor mediated vascular contraction. Eur. J. Pharmacol. 2000, 404, 361-368.

The following references show that 5-HT_{2A} agonism is associated with platelet aggregation, thrombosis and atherosclerosis:
(a) Li, N. et al. Effects of serotonin on platelet activation in whole blood. Blood Coagul Fibrinolysis 1997, 8, 517-523.
(b) Takano, S. Role of 5-hydroxytryptamine in platelet thrombus formation and mechanisms of inhibition of thrombus formation by 5-hydroxytryptamine 2A antagonists in rabbits. Arch. Int. Pharmacodyn. Ther. 1995, 330, 297-308.
(c) de Clerck, F. The role of serotonin in thrombogenesis. Clin. Physiol. Biochem. 1990, 8 (Suppl. 3), 40-49.

One of the initiating events of atherosclerois is endothelial injury followed by platelet aggregation. During atherosclerosis, platelets aggregate more readily and greater quantities of serotonin are released from platelets. Vascular responses to serotonin released by activated platelets are profoundly altered in a direction that favours vasoconstriction (arterial spasm), which ultimately may lead to thrombosis and complete obstruction of the vessel. Vascular smooth muscle cell proliferation is believed to play an important role in the pathogenesis of atherosclerosis. Serotonin is known to be a mitogen for vascular smooth muscle cells by stimulation of the 5-HT_{2A} receptor [tentatively via activation of MAPK (mitogen-activated protein kinase) and/or PKC (protein kinase C) dependent pathways], see:
(a) Lee, S.-L. et al. Serotonin stimulates mitogen-activated protein kinase activity through the formation of superoxide anion. Am. J. Physiol. 1999, 277, (2Pt.1), L-282-L291.
(b) Florian, J.A. et al. Integration of mitogen-activated protein kinase activation in vascular 5-hydroxytryptamine2A receptor signal transduction. J. Pharmacol. Exp. Ther. 1998, 284, 346-355.
(c) Banes A. et al. Mechanisms of 5-hydroxytryptamine2A receptor activation of the mitogen-activated protein kinase pathway in vascular smooth muscle. J. Pharmacol. Exp. Ther. 1999, 291,1179-1187.
(d) Watanabe, T. et al. Lipid peroxidation product 4-hydroxy-2-nonenal acts synergistically with serotonin in inducing vascular smooth muscle cell proliferation. Atherosclerosis 2001, 155,37-44.
(e) Pakala, R. et al. Eicosapentaenoic Acid and Docosahexaenoic Acid Block Serotonin-Induced Smooth Muscle Cell Proliferation. Atherioscler. Thromb. Vasc. Biol. 1999, 19, 2316-2322.

Furthermore, it has been suggested that increased vasoconstrictor response to 5-HT in atherosclerotic vessels might be due to supersensitive 5-HT_{2A} receptors, see: Fujiwara, T. et al. Augmented responses to 5-HT2-receptor-mediated vasoconstrictions in atherosclerotic rabbit common carotid arteries. J. Cardiovasc. Pharmacol. 1995, 26, 503-510.

A 5-HT-induced upregulation of thrombin receptor expression in vascular smooth muscle cells, via activation of 5-HT_{2A} receptors, has also been implicated to play a role in atherosclerosis, see: Schini-Kerth V.B. et al. Serotonin stimulates the expression of thrombin receptors in cultured vascular smooth muscle cells. Role of protein kinase C and protein tyrosine kinases. Circulation 1996, 93, 2170-2177.

The following references show that 5-HT_{2B} agonism is associated with migraine:
(a) Schmuck, K. et al. Activation of meningeal 5-HT2B receptors: an early step in the generation of migraine headache. Eur. J. Neurosci. 1996, 8, 959-967.
(b) Johnson, K.W. et al. Serotonin in migraine: Theories, animal models and emerging therapies. Prog. Drug. Res. 1998, 51, 219-244.
(c) Parsons, A.A. Prophylaxis of migraine. Curr. Opin. CPNS Invest. Drugs 2000, 2, 160-166.

The following reference show that pulmonary hypertension is associated with activation of the 5-HT_{2B} receptor:
(a) Launay, J.-M. et al. Function of the serotonin 5-hydroxytryptamine 2B receptor in pulmonary hypertension. Nature Med. 2002, 8,1129-1135.

The hollowing references show that 5-HT_{2A} and especially 5-HT_{2B} agonism is associated with the origin of cardiac fibrosis after treatment with marketed anti-obesity preparations such as dexfenfluramin:
(a) Rothman, R.B. et al. Evidence for possible involvment of 5-HT2B receptors in the cardiac valvulopathy associated with fenfluramine and other serotonergic medications. Circulation 2000, 102, 2836-2841.
(b) Fitzgerald, L.W. et al. Possible role of valvular serotonin 5-HT2B receptors in the cardiopathy associated with fenfluramine. Mol. Pharmacol. 2000, 57, 75-81.
(c) Setola, V. et al. 3,4-Methylenedioxymethamphetamine (MDMA, "Ecstasy") induces fenfluramine-like proliferative actions on human cardiac valvular interstitial cells in vitro. Mol. Pharmacol. 2003, 63, 1223-1229.

As denoted below, the present compounds may be used in order to treat serotonin-related conditions such as menopausal and post-menopausal hot flushes. Berendsen H.H.G. "Hot flushes and serotonin". Journal of the British Menopause Society. 2002, 8, 30-34, indicates that non-hormonal treatment with either 5-HT_{2A} receptor antagonists or 5-HT_{2C} receptor agonists may have several advantages over hormonal therapy.

Another example of serotonin-related disorders is weight gain associated with antipsychotic drug administration. WO 02/19998 discloses that the use of atypical antipsychotic agents is associated with weight gain in up to 50% of patients, a significant portion of the patient population. Piesla, M.J. et al. Atypical antipsychotic-like effects of 5-HT2C agonists. Schizophrenia Res. 2001, 49 (1-2; Sp Iss, Suppl.) 95, discloses that 5-HT_{2C} agonists have anti-psychotic potential. Therefore, therapy using 5-HT_{2C} agonists will probably counteract an increase in body weight induced by antipsychotic drugs without counteracting the antipsychotic effect. It is reasonable to expect that the antipsychotic effect will be strengthened.

### Summary of the invention

According to the present invention, a class of novel compounds have been developed which bind to the 5-HT_{2C} receptor, which compounds may be agonists, partial agonists or antagonists for this receptor, preferably agonists or partial agonists. Therefore, the present compounds may be used for the treatment of serotonin-related disorders and conditions. Furthermore, it has been shown that the 5-HT_{2C} receptor selectivity of the present compounds is unexpectedly high compared to prior art compounds.

In one aspect, the invention provides novel compounds of the general Formula (I): 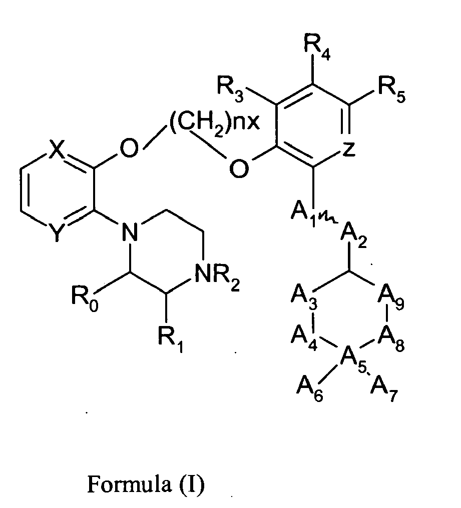 wherein
nx is 2-4, preferably 2;
R₀ and R₁ are each independently H or CH₃;
R₂ is H, C₁-C₄-alkyl, 2-hydroxyethyl, 2-cyanoethyl or tetrahydropyran-2-yl, C₁-C₄-acyl or C₁-C₄-alkoxycarbonyl;
R₃-R₅ are each independently H, halogen, methyl or methoxy, provided that at least one of R₃-R₅ is hydrogen;
X, Y, and Z are each independently CH or N;
A₁ is O, CH or CH₂;
A₂ is O, CH or (CH₂)ₙ₂, wherein n2 is an integer 0-2;
the bond between A₁ and A₂ is a single or double bond;
A₃ is (CH₂)ₙ₃, wherein n3 an integer 0-10, preferably 0-7, more preferably 0-5;
A₄ is (CMe₂)ₙ₄, wherein n4 is an integer 0-1;
A₅ is N or O;
A₆ and A₇ are each independently H, C₁-C₄-alkyl, amino-C₂-C₄-alkyl, N,N-di-C₁-C₄-alkylamino-C₂-C₄-alkyl, C₃-C₆-cycloalkyl or form together with A₅ a saturated heterocyclic ring;
A₈ is (CH₂)ₙ₈, wherein n8 is an integer 0-2;
A₉ is CH₂;
or pharmaceutically acceptable salts, solvates, hydrates, geometrical isomers, tautomers, optical isomers, and N-oxides thereof;
provided that when A₅ is N, then A₅ is substituted by only two of A₆, A₇ and A₈; when A₅ is O, then A₅ is substituted by only one of A₆, A₇ and A₈.

In case the compounds of Formula (I) can be in the form of optical isomers, the invention comprises the racemic mixture as well as the individual enantiomers as such.

In other aspects, the compounds of formula (I) are those wherein:

In case at least one of the integers n2, n3, n4 or n8 referred to above equals 0, then the corresponding group A₂, A₃, A₄ or A₈ equals a single bond. In the case when n8 equals 1 or 2, then A₅ is not substituted by A₇.

When A₁ is CH₂, A₂ is O and A₅ is N, then both n3 and n4 are not 0.

When one of A₁ and A₂ is O, the other of A₁ and A₂ may not be O.

When one of A₁ and A₂ is CH, then the other of A₁ and A₂ is also CH, wherein the bond between A₁ and A₂ is a double bond.

When A₃, A₄, A₅, A₈ and A₉ together with the carbon atom between A₃ and A₉ form a 4-7 membered saturated heterocyclic ring (e.g. azacyclic ring), then A₉ is CH₂, n4 is 0, and the sum of n3 + n8 is an integer 1-4.

When A₉ is CH₂, then all of n3, n4, and n8 are not 0.

When A₉ is CH₂ and A₅ is N, then n4 is 0.

When A₅ is nitrogen, the A₆ and A₇ are each independently H, C₁-C₄-alkyl, C₃-C₆-cycloalkyl or form together a saturated heterocyclic ring.

In case R₀ is methyl, it is preferred that the carbon atom, to which the said methyl group R₀ is attached, is in the (R)-configuration.

It is preferred that R₁ is hydrogen.

It is also preferred that X and Y both are nitrogen.

It is also preferred that R₂ is H or methyl.

It is also preferred that all of R₃-R₅ are H.

It is also preferred that A₆ and A₇ are each independently H, methyl, isopropyl, 2-ethylamine or form together with A₅ a pyrrolidine or piperazine ring.

In one preferred embodiment, the invention refers to a compound of Formula (Ib): 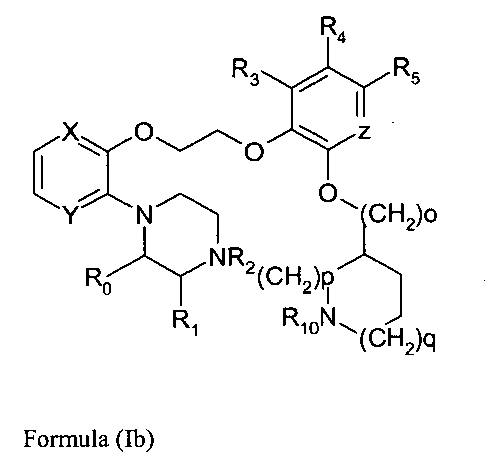 wherein:
R₀-R₅, X, Y, and Z are as defined above,
o is an integer 0-2;
p is an integer 0-2, wherein o and p are preferably not both 0;
q is an integer 0-1;
R₁₀ is H or C₁-C₄-alkyl, preferably H or methyl.

Specific compounds of Formula (Ib) are:
2-[(2R)-2-Methylpiperazin-1-yl]-3-[2-({2-[(1-methylpiperidin-4-yl)oxy]pyridin-3-yl} oxy)ethoxy]pyrazine;
2-[(2R)-2-Methylpiperazin-1-yl]-3-[2-({2-[2-(1-methylpyrrolidin-2-yl)ethoxy]pyridin-3-yl}oxy)ethoxy]pyrazine;
2-[(2R)-2-Methylpiperazin-1-yl]-3-(2-{[2-(piperidin-3-ylmethoxy)pyridin-3-yl]oxy} ethoxy)pyrazine;
2-[(2R)-2-Methylpiperazin-1-yl]-3-{2-[(2- {[(2*S*)-1-methylpyrrolidin-2-yl]methoxy}pyridin-3-yl)oxy]ethoxy} pyrazine.

The compounds of each of Formula (I) and (Ib) are collectively referred to as those of Formulae (I).

In case the compounds of Formulae (I) contain groups which may exist in tautomeric forms, the invention comprises the tautomeric forms of the compounds as well as mixtures thereof.

In case the compounds of Formulae (I) can be in the form of geometrical isomers, the invention comprises the geometrical isomers as well as mixtures thereof.

In another aspect, the invention provides the compounds according to Formulae (I) above for use in therapy of a human being or an animal.

Still another aspect of the invention provides a pharmaceutical composition comprising a compound according to Formulae (I) above as the active ingredient, preferably together with a pharmaceutically acceptable carrier and, if desired, other pharmacologically active agents.

In yet another aspect, the invention provides a method for the treatment of a human or animal subject suffering from a serotonin-related disorder or condition, particularly 5-HT_{2C} receptor-related, such as memory disorders, such as Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

Another aspect of the invention provides the use of the compounds according to Formulae (I) above for the manufacture of a medicament for the treatment of a serotonin-related disorder or condition, particularly 5-HT_{2C} receptor-related, such as memory disorders, such as Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity. The method can include administering to a subject (e.g., a human or an animal) in need thereof an effective amount of one or more compounds of Formulae (I), their salts, or compositions containing the compounds or salts.

Another aspect of the invention provides methods for modulating 5-HT_{2C} receptor function comprising contacting the receptor with an effective stimulatory or inhibitory amount of a compound according to Formulae (I) above, preferably an effective stimulatory amount thereof.

The methods delineated herein can also include the step of identifying that the subject is in need of treatment of the serotonin-related disorder or condition. Identifying a subject in need of such treatment can be in the judgment of a subject or a health care professional and can be subjective (e.g., opinion) or objective (e.g., measurable by a test or diagnostic method).

This invention also features a method for preparing a composition. The method includes combining a compound of Formulae (I) with a pharmaceutically acceptable carrier.

Still another aspect of the invention provides methods for the preparation of the compounds according to Formulae (I) above.

A further aspect of the invention relates to a method for treating a disorder or condition, comprising administering to a subject in need thereof an effective amount of a compound of Formulae (I) above, wherein the disorder or condition is selected from memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

A still further aspect of the invention relates to the use of the compounds of Formulae (I) above for the manufacture of a medicament for the treatment of memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

### Detailed description of the invention

First, the various terms used, separately and in combinations, in the above definition of the compounds having the general Formula (I) (and each of Formulae (I))will be explained.

C₁-C₄-alkyl, which may be straight or branched, is an alkyl group having 1-4 carbon atoms. Exemplary alkyl groups include methyl, ethyl, n-propyl, isopropyl, n-butyl, sec-butyl, isobutyl, and tert-butyl.

Amino-C₂-C₄-akyl, which may be straight or branched, is a C₂-C₄-alkyl group directly attached to an amino group. Exemplary aminoalkyl groups include 2-aminoethyl, 3-amino-n-propyl, and 4-amino-n-butyl.

C₃-C₆-cycloalkyl is a cyclic alkyl group having 3-6 carbon atoms. Exemplary cycloalkyl groups are cyclopropyl, cyclobutyl, cyclopentyl, and cyclohexyl.

C₁-C₄-acyl, which may be straight or branched, is an acyl group having 1-4 carbon atoms. Exemplary acyl groups include formyl, acetyl, propionyl, n-butyryl, and isobutyryl.

C₁-C₄-alkoxy, which may be straight or branched, is an alkoxy group having 1-4 carbon atoms. Exemplary alkoxy groups include methoxy, ethoxy, n-propoxy, isopropoxy, n-butoxy, sec-butoxy, isobutoxy, and tert-butoxy.

C₁-C₄-alkoxycarbonyl means a C₁-C₄-alkoxy group directly connected to a carbonyl group. An exemplary alkoxycarbonyl group is tert-butoxycarbonyl (t-BOC).

DCM means dichloromethane, DMSO means dimethylsulfoxide, halogen means fluoro, chloro, bromo, or iodo, HOAc means acetic acid, HPLC means high performance liquid chromatography, HRMS means high resolution mass spectrometry, "OTs" means tosylate i.e. para-toluenesulfonate, "OMs" means mesylate i.e. methanesulfonate, TEA means triethylamine, TFA means trifluoroacetic acid, THF means tetrahydrofuran.

The term "saturated heterocyclic" refers to a heterocyclic ring that is non-aromatic (e.g., partially or fully saturated) and that has carbon atoms and one or two heteroatoms selected from O, S, and N (preferably from O and N and more preferably from N). Examples of saturated heterocyclic rings have 4-7 members and include piperidine, azetidine, morpholine, thiomorpholine, pyrrolidine, and piperazine.

Hydrogenation catalyst means a catalyst suitable for catalytic hydrogenation ordebenzylation. Examples of hydrogenation catalysts are palladium on carbon (Pd/C), Raney-Nickel, platinum, platinum oxide and zinc oxide.

Hydrogen source means a reagent used to introduce a hydrogen atom on any atom of a compound, including a carbon or oxygen atom. Examples of hydrogen sources are hydrogen gas and ammonium formate.

Hydroxyethylating agent means a reagent used to introduce a hydroxyethyl group on an oxygen or nitrogen atom of a compound. Examples of hydroxyethylating agents are ethylene carbonate, 2-bromoethanol, 2-chloroethanol and ethylene oxide.

For the transformation of an alcohol function into an aldehyde function, the method by Swern et al., J. Org. Chem. 1978, 43, 2480-2482, may be used. According to this method, the alcohol is reacted with dimethyl sulfoxide and oxalyl chloride in dichloromethane at a temperature of -78°C.

For the transformation of an alcohol function into a suitable leaving group, one may treat the alcohol with methanesulfonic anhydride in the presence of triethylamine in dichloromethane at a temperature of 0°C, e.g. as disclosed in J. Org. Chem. 2000, 65, 7839-7846.

A base is any substance that produces a negative ion and denotes electrons to an acid, if present. The term "base" as used herein, represents a reagent capable of accepting protons during the course of a reaction. Examples of bases include carbonate salts such as potassium carbonate, potassium bicarbonate, sodium carbonate, sodium bicarbonate, and cesium carbonate; halides such as cesium fluoride; phosphates such as potassium phosphate, potassium dihydrogen phosphate, and potassium hydrogen phosphate; hydroxides such as lithium hydroxide, sodium hydroxide, and potassium hydroxide; alkoxides such as sodium tert-butoxide, potassium tert-butoxide, and lithium tert-butoxide; alkylamines such as triethylamine, diisopropylamine, and diisopropylethylamine; heterocyclic amines such as 4-dimethylaminopyridine, 2,6-lutidine, 1-methylimidazole, pyridine; bicyclic amines such as 1,8-diazabicyclo(4.3.0)undec-7-ene; and hydrides such as lithium hydride, sodium hydride, and potassium hydride. The base chosen for a particular conversion depends on the nature of the starting materials, the solvent or solvents in which the reaction is conducted, and the temperature at which the reaction is conducted.

The term "prodrug forms" means a pharmacologically acceptable derivative, such as an ester or an amide, which derivative is biotransformed in the body to form the active drug. Reference is made to Goodman and Gilman's, The Pharmacological basis of Therapeutics, 8th ed., McGraw-Hill, Int. Ed. 1992, "Biotransformation of Drugs, p. 13-15; and "The Organic Chemistry of Drug Design and Drug Action" by Richard B. Silverman. Chapter 8, p 352. (Academic Press, Inc. 1992. ISBN 0-12-643730-0).

"Pharmaceutically acceptable" means being useful in preparing a pharmaceutical composition that is generally safe, non-toxic and neither biologically nor otherwise undesirable and includes being useful for veterinary use as well as human pharmaceutical use.

"Pharmaceutically acceptable salts" mean salts which are pharmaceutically acceptable, as defined above, and which possess the desired pharmacological activity. Such salts include acid addition salts formed with organic and inorganic acids, such as hydrogen chloride, hydrogen bromide, hydrogen iodide, sulfuric acid, phosphoric acid, acetic acid, glycolic acid, maleic acid, malonic acid, malic acid, oxalic acid, toluenesulphonic acid, methanesulphonic acid, trifluoroacetic acid, fumaric acid, succinic acid, tartaric acid, citric acid, benzoic acid, ascorbic acid, isethionic acid (i.e. 2-hydroxyethylsulphonic acid) and the like.

"Treatment" as used herein includes prophylaxis of the named disorder or condition, or amelioration or elimination of the disorder or condition once it has been established.

It should be noted that both E- and Z-isomers of the compounds, optical isomers, as well as mixtures thereof, and all isotopes are included within the scope of the invention. E means "entgegen" (trans-isomer) and Z means "zusammen" (cis-isomer).

Presently preferred compounds of the general Formula (I) above are the compounds according to Examples 3-6; and their pharmacologically acceptable salts and solvates.

As mentioned above, the compounds of the present invention are useful for the treatment of a human or animal subject suffering from a serotonin-related disorder or condition, particularly 5-HT_{2C} receptor-related, such as memory disorders, such as Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity. The 5-HT_{2C} receptor-related disorder includes any disorder or condition that is modulated by the 5-HT_{2C} receptor. Preferably, the compounds of the present invention may be used in the treatment of disorders and conditions where a 5-HT_{2C} receptor agonist is desired or required.

The compounds of the present invention in labelled form, e.g. isotopically labelled, may be used as a diagnostic agent. Examples of such labels are known in the art and include ¹³¹I, ³⁵S, ³²P, ¹⁸F, ¹⁴C, ¹¹C, ³H, and the like.

Another object of the present invention is a process for the preparation of a compound of the Formula (Ib), which process comprises:
a) reacting a compound of Formula (II) 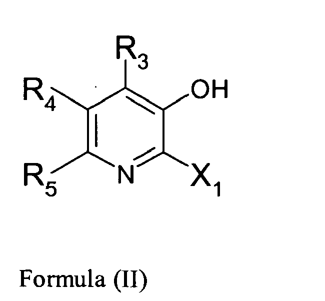 wherein:
   X₁ is selected from F, Cl, Br and I,
   R₃-R₅ are each independently H, halogen, methyl or methoxy, provided that at least one of R₃-R₅ is hydrogen,
   with a compound of Formula (III): 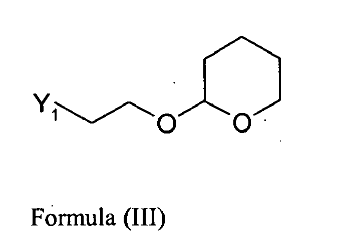
   wherein Y₁ is a suitable leaving group selected from Cl, Br, I, OTs, or OMs;
   in the presence of a base, such as potassium carbonate, triethylamine, or pyridine, in a solvent, such as acetonitrile, to give a compound of Formula (IV): 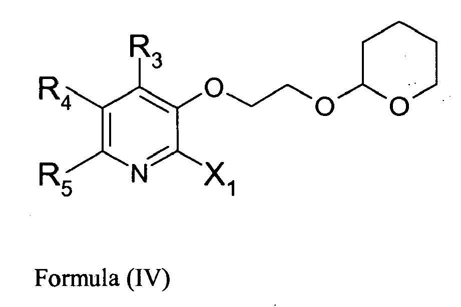 wherein X₁ and R₃-R₅ are as defined above;
b) reacting the compound of Formula (IV) with a compound of Formula (Vb) in the presence of a base, such as potassium tert-butoxide, in a solvent, such as toluene, 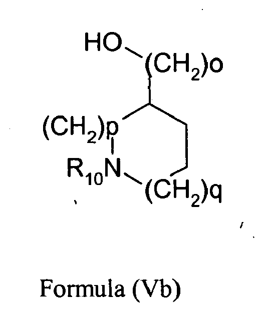 wherein:
   o is an integer 0-2;
   p is an integer 0-2, wherein o and p are preferably not both 0;
   q is an integer 0-1;
   R₁₀ is H or C₁-C₄-alkyl, preferably H or methyl;
   to give a compound of Formula (VIb): 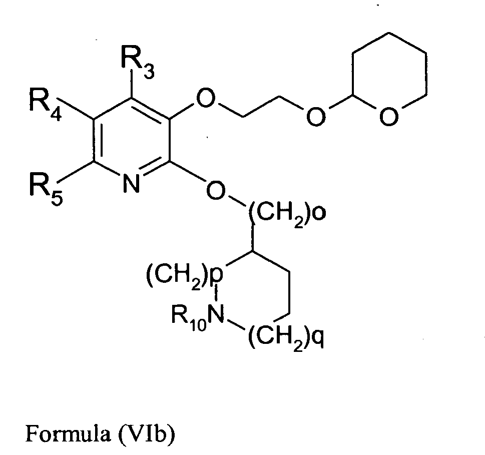 wherein:
   R₃-R₅ and R₁₀, o, p, and q are as defined above,
c) treating the compound of Formula (VIb) with an aqueous acid such as aqueous acetic acid or aqueous hydrochloric acid, to give a compound of Formula (VIIb): 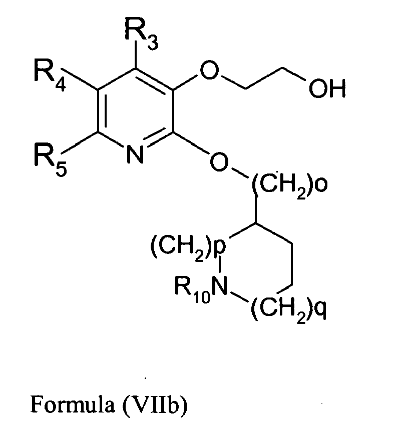 wherein:
   R₃-R₅ and R₁₀, o, p, and q are as defined above,
d) reacting the compound of Formula (VIIb) with a compound of Formula (VIII) in the presence of a base such as potassium tert-butoxide, in a solvent, such as methyl tert-butyl ether or toluene, 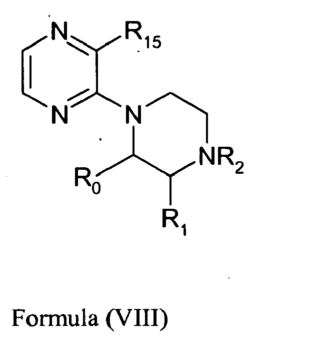 wherein
   R₀ and R₁ are each independently H or CH₃;
   R₂ is selected from C₁-C₄-alkoxycarbonyl, benzyl, trityl, C₁-C₄-alkyl, 2-hydroxyethyl, 2-cyanoethyl, tetrahydropyran-2-yl, and C₁-C₄-acyl,
   R₁₅ is halogen, such as chlorine, to give a compound of Formula (Ib) wherein X = N and Y = N: 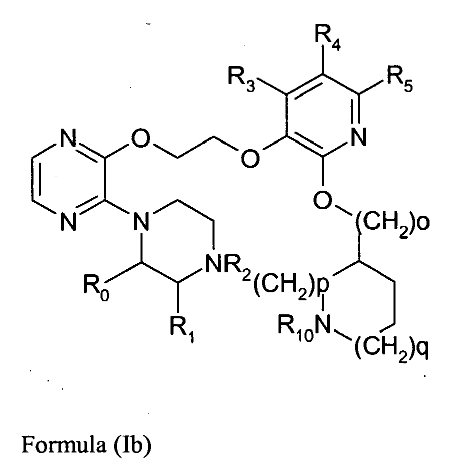 wherein R₀-R₅, R₁₀, o, p, and q are as defined above;
e) if desired, separating a racemate obtained into optical isomers and/or forming an acid addition salt with an organic or inorganic acid,
f) if R₂ in Formula (Ib) following step d) is a nitrogen protecting group, such as t-BOC, trityl, and benzyl, removing said nitrogen protecting group, such as under acidic conditions (e.g. trifluoroacetic acid in a solvent such as chloroform), hydrogenolytic or non-hydrogenolytic conditions, to provide the compound of Formula (Ib), wherein R₂ is hydrogen.

Another object of the present invention is a process for the preparation of a compound of the Formula (Ib), which process comprises:
a) reacting a compound of Formula (IX) with a benzylating agent, such as benzyl chloride, benzyl bromide, or benzyl tosylate, in the presence of a base: 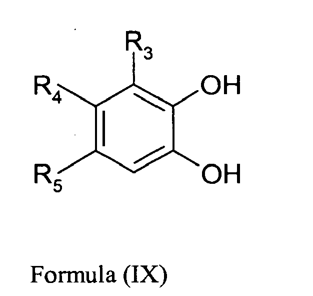 wherein R₃-R₅ are each independently H, halogen, methyl, or methoxy, provided that at least one of R₃-R₅ is hydrogen, to give a compound of Formula (X): 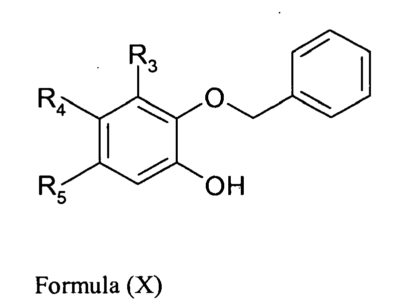 wherein R₃-R₅ are as defined above,
b) reacting the compound of Formula (X) with a compound of Formula (XIb) in the presence of a base, such as potassium carbonate, in a solvent, such as acetone: 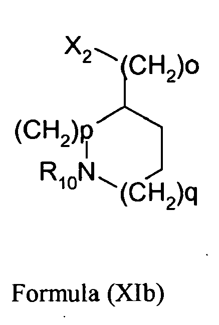 wherein:
   X₂ is halogen, OMs, or OTs;
   o is an integer 0-2;
   p is an integer 0-2, wherein o and p are preferably not both 0;
   q is an integer 0-1;
   R₁₀ is H or C₁-C₄-alkyl, preferably H or methyl;
   to give a compound of Formula (XIIb): 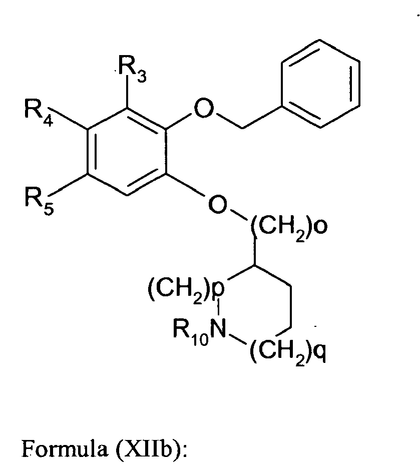 wherein:
   R₃-R₅, R₁₀, o, p, and q are as defined above,
c) treating the compound of Formula (XIIb) with hydrogen in the presence of a hydrogenation catalyst, such as palladium on carbon, in a solvent, such as methanol, to give a compound of Formula (XIIIb): 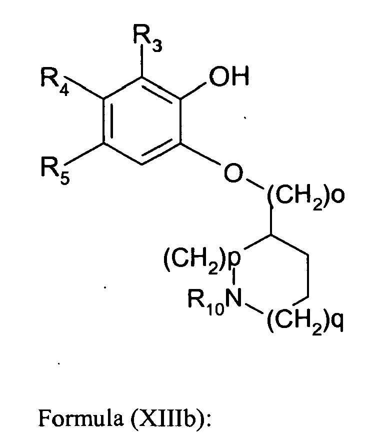 wherein:
   R₃-R₅, R₁₀, o, p, and q are as defined above,
d) reacting the compound of Formula (XIIIb) with a hydroxyethylating agent such as ethylene carbonate in the presence of a base, such as potassium carbonate, in a solvent, such as N,N-dimethylformamide, to give a compound of Formula (XIVb): 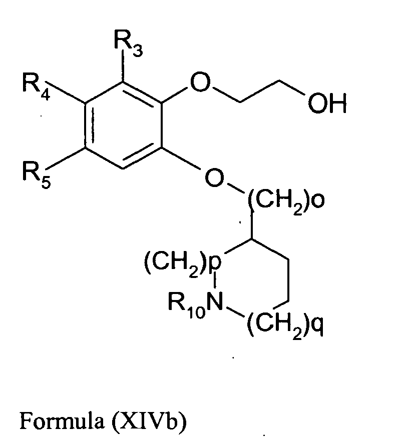 wherein R₃-R₅, R₁₀, o, p, and q are as defined above;
e) reacting the compound of Formula (XIVa) with a compound of Formula (VIII) in the presence of a base, such as potassium tert-butoxide, in a solvent, such as N,N-dimethylformamide, 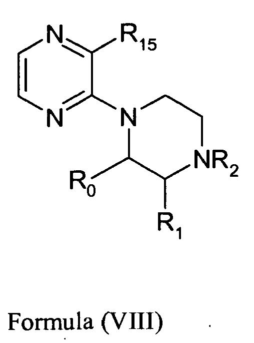 wherein
   R₀ and R₁ are each independently H or CH₃;
   R₂ is selected from C₁-C₄-alkoxycarbonyl, benzyl, trityl, C₁-C₄-alkyl, 2-hydroxyethyl, 2-cyanoethyl, tetrahydropyran-2-yl, and C₁-C₄-acyl,
   R₁₅ is halogen, such as chlorine, to give a compound of Formula (Ib) wherein X = N and Y = N: 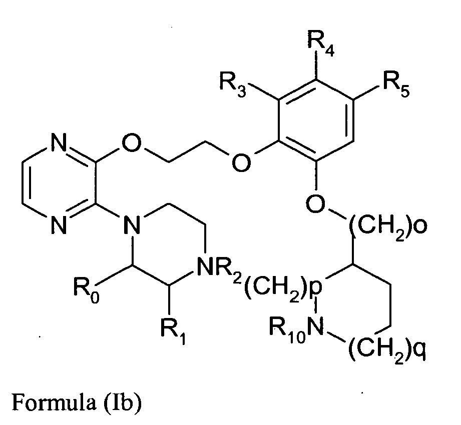 wherein R₀-R₅, R₁₀, o, p, and q are as defined above;
f) if desired, separating a racemate obtained into optical isomers and/or forming an acid addition salt with an organic or inorganic acid,
g) if R₂ in Formula (Ib) following step e) is a nitrogen protecting group, such as t-BOC, trityl, and benzyl, removing said nitrogen protecting group, such as under acidic conditions (e.g., trifluoroacetic acid in a solvent such as chloroform), hydrogenolytic or non-hydrogenolytic conditions, to provide the compound of Formula (Ib), wherein R₂ is hydrogen.

Another object of the present invention is a process for the preparation of a compound of the Formula (Ib), which process comprises:
a) reacting a compound of Formula (XV) with a compound selected from benzyl chloride, benzyl bromide, benzyl iodide, benzyl tosylate, and benzyl mesylate in the presence of a base, such as potassium carbonate, in a solvent, such as N,N-dimethylformamide, 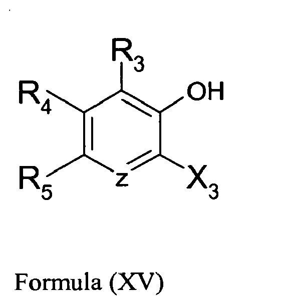 wherein:
   X₁ is selected from Cl, Br and I, Z is CH or N, R₃-R₅ are each independently H, halogen, methyl or methoxy, provided that at least one of R₃-R₅ is hydrogen, to give a compound of Formula (XVI): 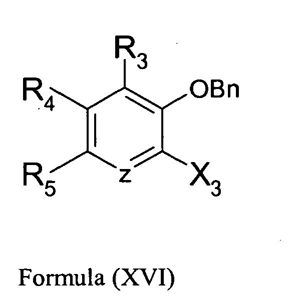 wherein R₃-R₅, X₃, and Z are as defmed above;
b) reacting the compound of Formula (XVI) with a compound of Formula (Vb) in the presence of a base, such as sodium tert-butoxide, in a solvent, such as N,N-dimethylformamide, 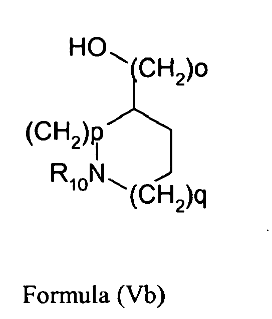 wherein:
   o is an integer 0-2;
   p is an integer 0-2, wherein o and p are preferably not both 0;
   q is an integer 0-1;
   R₁₀ is H or C₁-C₄-alkyl, preferably H or methyl;
   to give a compound of Formula (XVIIb): 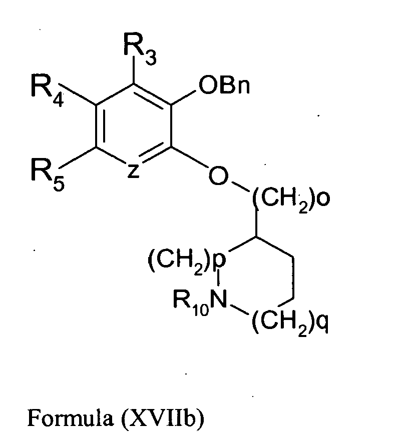 wherein:
   R₃-R₅ and R₁₀, o, p, q, and Z are as defined above,
c) treating the compound of Formula (XVIIb) with a hydrogenation catalyst using a suitable hydrogen source such as ammonium formate and then heating in the presence of a hydroxyethylating agent, preferably ethylene carbonate, and a base, such as potassium carbonate, in a solvent, such as N,N-dimethylformamide, to give a compound of Formula (VIIb): 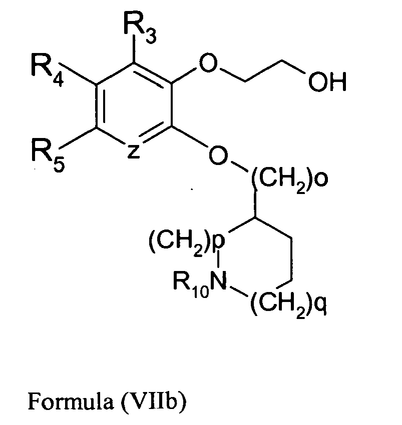 wherein:
   R₃-R₅ and R₁₀, o, p, q, and Z are as defined above,
d) reacting the compound of Formula (VIIb) with a compound of Formula (VIII) in the presence of a base such as sodium tert-butoxide, in a solvent, such as N,N-dimethylformamide, 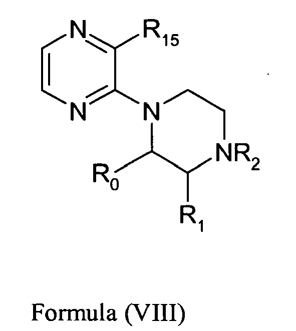 wherein
   R₀ and R₁ are each independently H or CH₃;
   R₂ is selected from C₁-C₄-alkoxycarbonyl, benzyl, trityl, C₁-C₄-akyl, 2-hydroxyethyl, 2-cyanoethyl, tetrahydropyran-2-yl, and C₁-C₄-acyl,
   R₁₅ is halogen, such as chlorine, to give a compound of Formula (Ib) wherein X = N and Y = N: 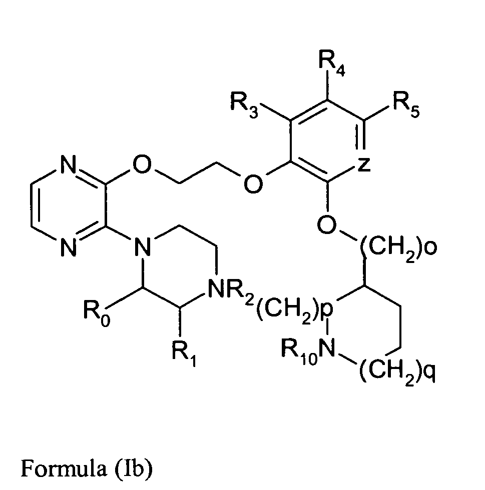 wherein R₀-R₅, R₁₀, o, p, q, and Z are as defined above;
e) if desired, separating a racemate obtained into optical isomers and/or forming an acid addition salt with an organic or inorganic acid,
f) if R₂ in Formula (Ib) following step d) is a nitrogen protecting group, such as t-BOC, trityl, and benzyl, removing said nitrogen protecting group, such as under acidic conditions (e.g. trifluoroacetic acid in a solvent such as chloroform), hydrogenolytic or non-hydrogenolytic conditions, to provide the compound of Formula (Ib), wherein R₂ is hydrogen.

The chemicals used in the above-described synthetic routes may include, for example, solvents, reagents, catalysts, protecting group and deprotecting group agents. The methods described above may also additionally include steps, either before or after the steps described specifically herein, to add or remove suitable protecting groups in order to ultimately allow synthesis of the compounds of Formulae (I). In addition, various synthetic steps may be performed in an alternate sequence or order to give the desired compounds. Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof. Examples of protecting groups according to the present invention are t-BOC (tert-butoxycarbonyl), trityl, and benzyl.

It should be noted that a compound of Formula (I) (including any of Formula Ib) as prepared according to the present invention may be converted to another compound of Formula (I) by methods well known in the art. For example, a standard reductive alkylation reaction can be the preparation of a compound of Formula (I) wherein R₂ is methyl from the corresponding compound of Formula (I) wherein R₂ is hydrogen (cf. the protocol described in J. Org. Chem. 1996, 61, 3849-3862).

The processes described above may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are maleic acid, fumaric acid, succinic acid, methanesulfonic acid, trifluoroacetic acid, acetic acid, oxalic acid, benzoic acid, malic acid, hydrochloric acid, sulphuric acid, phosphoric acid, isethionic acid, and the like.

The compounds of Formulae (I) may possess one or more chiral carbon atoms, and they may therefore be obtained in the form of optical isomers, e.g. as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

The necessary starting materials for preparing the compounds of Formulae (I) are either known or may be prepared in analogy with the preparation of known compounds.

In accordance with the present invention, the compounds of Formulae (I), in the form of free bases or salts with physiologically acceptable acids, can be brought into suitable galenic forms, such as compositions for oral use, for injection, for nasal spray administration or the like, in accordance with accepted pharmaceutical procedures. Such pharmaceutical compositions according to the invention comprise an effective amount of the compounds of Formulae (I) in association with compatible pharmaceutically acceptable carrier materials, or diluents, as are well known in the art. The carriers may be any inert material, organic or inorganic, suitable for enteral, percutaneous, subcutaneous or parenteral administration, such as: water, gelatin, gum arabicum, lactose, microcrystalline cellulose, starch, sodium starch glycolate, calcium hydrogen phosphate, magnesium stearate, talcum, colloidal silicon dioxide, and the like. Such compositions may also contain other pharmacologically active agents, and conventional additives, such as stabilizers, wetting agents, emulsifiers, flavouring agents, buffers, and the like.

The compositions according to the invention can e.g. be made up in solid or liquid form for oral administration, such as tablets, pills, capsules, powders, syrups, elixirs, dispersable granules, cachets, suppositories and the like, in the form of sterile solutions, suspensions or emulsions for parenteral administration, sprays, e.g. a nasal spray, transdermal preparations, e.g. patches, and the like.

As mentioned above, the compounds of the invention may be used for the treatment of a human or animal subject suffering from a serotonin-related disorder or condition, particularly 5-HT_{2C} receptor-related, such as memory disorders, such as Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

This invention also relates to a method of treatment or prophylaxis of a serotonin-related disorder or condition as described above. The method includes administering to a subject (e.g., a human, a horse, a dog, or a cat) in need thereof an effective amount of one or more compounds of Formulae (I) described above. The methods delineated herein can also include the step of identifying that the subject is in need of treatment of the serotonin-related disorder or condition.

Also within the scope of this invention is a method for modulating (e.g., stimulating or inhibiting) 5-HT_{2C} receptor function. The method includes contacting the receptor with an effective stimulatory or inhibitory amount of a compound of Formula (I), preferably an effective stimulatory amount thereof. The contacting step can include administering a compound, its salt, or a composition containing the compound or the salt.

"An effective amount" refers to an amount of a compound which confers a therapeutic effect on the treated subject. The therapeutic effect may be objective (i.e., measurable by some test or marker) or subjective (i.e., subject gives an indication of or feels an effect). The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

The invention will now be further illustrated by the following non-limiting Examples. Without further elaboration, it is believed that one skilled in the art can, based on the description herein, utilize the present invention to its fullest extent. All publications cited herein are hereby incorporated by reference in their entirety.

### EXAMPLES

### General.

Unless otherwise noted, starting materials were obtained from commercial sources and used as received. ¹H nuclear magnetic resonance (NMR) and¹³C NMR spectra were recorded on a Bruker Advance DPX 400 spectrometer at 400.1 and 100.6 MHz, respectively, or on a JEOL 270 spectrometer. All spectra were recorded using residual solvent as internal standard. Melting points were determined with a Koefler bench and are uncorrected. Electrospray mass spectrometry (MS) spectra were obtained on a Perkin-Elmer API 150EX mass spectrometer. Accurate mass measurements were performed on a Micromass LCT dual probe.

HPLC conditions for Examples 1-6: Preparative HPLC was performed on a Gilson-system equipped with an YMC AQ C18, 5 µm column (20x50mm), eluent: water/acetonitrile + 0.1% trifluoroacetic acid. Analytical LC-UV was performed on an Agilent 1100 system with an ACE C8, 3 µm column (3x50 mm), eluent: water/acetonitrile + 0.1% trifluoroacetic acid. Analytical LC-MS was performed on an Agilent 1100 system with an YMC AQ C18, 3 µm column (3x33 mm), eluent: water/acetonitrile +0,1% trifluoroacetic acid.

### EXAMPLE 1 (intermediate)

### 2-Chloro-3-[2-(tetrahydro-2H-pyran-2-yloxy)ethoxy]pyridine.

A mixture of 2-chloro-3-hydroxypyridine (5.00 g, 38.6 mmol), 2-(2-bromoethoxy)tetrahydro-2-pyran (5.85 mL, 38.6 mmol) and potassium carbonate (6.40 g, 46.3 mmol) in 200 mL of acetonitrile was heated at reflux overnight. The reaction mixture was filtered, concentrated under reduced pressure and redissolved in 300 mL of ethyl acetate. The organic phase was washed with 3 x 100 mL of NaOH and 100 mL of brine, dried (MgSO₄) and concentrated under reduced pressure. The residue was purified with flash chromatography on a column of silica to give 7.21 g (28.0 mmol, 73%) of the title compound as a yellow oil. LC-UV purity 100%. MS m/z 258 (M+1)⁺, calc. 258 (M+1)⁺.

### EXAMPLE 2 (intermediate)

### tert-Butyl (3R)-4-(3-chloropyrazin-2-yl)-3-methylpiperazine-1-carboxylate.*

To a solution of 2-chloro-3-[(2R)-2-methylpiperazin-1-yl]pyrazine** (1.59 g, 7.48 mmol) in 200 mL of acetonitrile was boc-anhydride (1.63 g, 7.48 mmol) added in portions over 1 hour. The solution was stirred at room temperature overnight, quenched with 10 mL of water and concentrated. The residue was redissolved in 200 mL of ethyl acetate, washed with 100 mL of 1.0 M K₂CO₃, 100 mL of water and 100 mL of brine, dried (MgSO₄) and concentrated to give 1.70 g (5.43 mmol, 73%) of the title compound as a colorless oil. LC-UV purity 95%. MS *m*/*z* 313 (M+1)⁺, calc. 313 (M+1)⁺. *Reported in WO 00/76984, Example 172, Step 2. **Reported in WO 00/76984, Example 192, Step 2.

### EXAMPLE 3

### 2-[(2R)-2-Methylpiperazin-1-yl]-3-[2-({2-[(1-methylpiperidin-4-yl)oxy]pyridin-3-yl}oxy)ethoxy]pyrazine, trifluoroacetate.

### Step 1: 2-({2-[(1-Methylpiperidin-4-yl)oxy]pyridin-3-yl}oxy)ethanol.

A solution of 2-chloro-3-[2-(tetrahydro-2*H*-pyran-2-yloxy)ethoxy]pyridine (from Example 1; 100 mg, 0.39 mmol), 4-hydroxy-N-methylpiperidine (67 µl, 0.58 mmol) and 1.0 M potassium *tert*-butoxide in *tert*-butanol (0.8 mL, 0.80 mmol) in 4 mL of toluene was heated at 100°C for 1 day. The organic phase was washed with 3 x 2 mL of water and 2 mL of brine. The organic phase was shaken at 50 °C with 4 mL of 2.0 M acetic acid for 2 days. The aqueous phase was washed with 3 x 3mL of ethyl acetate, made basic by addition of potassium hydroxide, saturated with sodium chloride and extracted with 3 x 2 mL of ethyl acetate. The organic phase was dried (MgSO₄) and concentrated under reduced pressure to give 62 mg (0.25 mmol, 63%) of the title compound as a colorless oil. LC-UV purity 95%. MS *mlz* 253 (M+1)⁺, calc. 253 (M+1)⁺.

### Step 2: 2-[(2R)-2-Methylpiperazin-1-yl]-3-[2-({2-[(1-methylpiperidin-4-yl)oxy]pyridin-3-yl}oxy)ethoxy]pyrazine, trifluoroacetate.

To a solution of 2-({2-[(1-methylpiperidin-4-yl)oxy]pyridin-3-yl}oxy)ethanol (from Step 1; 30 mg, 0.12 mmol) and 1.0 M potassium *tert*-butoxide in *tert*-butanol (0.15 mL, 0.15 mmol) in 4 mL of dry toluene was *tert*-butyl (3*R*)-4-(3-chloropyrazin-2-yl)-3-methylpiperazine-1-carboxylate (from Example 2; 31 mg, 0.10 mmol) in 1 mL of toluene added. The mixture was shaken at room temperature for 2 days. The reaction mixture was quenched with 2 mL of water. The organic phase was washed with 3 x 2 mL of water and 2 mL of brine and concentrated under reduced pressure. The residue was purified with reversed phase chromatography. The purified product was concentrated, redissolved in 1 mL of chloroform and treated with 0.2 mL of trifluoroacetic acid for 1 hour. The solvent was removed under reduced pressure to give 36 mg (0.066 mmol, 66%) of the title compound as a brown oil. LC-UV purity 100%. HRMS *mlz* calcd for C₂₂H₃₂N₆O₃ (M)⁺ 428.2536, found 428.2546.

### EXAMPLE 4

### 2-[(2R)-2-Methylpiperazin-1-yl]-3-[2-({2-[2-(1-methylpyrrolidin-2-yl)ethoxy]pyridin-3-yl}oxy)ethoxy]pyrazine, trifluoroacetate.

### Step 1: 2-({2-[2-(1-Methylpyrrolidin-2-yl)ethoxy]pyridin-3-yl}oxy)ethanol.

A solution of 2-chloro-3-[2-(tetrahydro-2*H*-pyran-2-yloxy)ethoxy]pyridine (from Example 1; 100 mg, 0.39 mmol), 1-methyl-2-pyrrolidineethanol (79 µl, 0.58 mmol) and 1.0 M potassium *tert*-butoxide in *tert*-butanol (0.8 mL, 0.80 mmol) in 4 mL of toluene was heated at 100 °C for 1 day. The organic phase was washed with 3 x 2 mL of water and 2 mL of brine. The organic phase was shaken at 50 °C with 4 mL of 2.0 M acetic acid for 2 days. The aqueous phase was washed with 3 x 3 mL of ethyl acetate, made basic by addition of potassium hydroxide, saturated with sodium chloride and extracted with 3 x 2 mL of ethyl acetate. The organic phase was dried (MgSO₄) and concentrated under reduced pressure to give 55 mg (0.21 mmol, 53%) of the title compound as a colorless oil. LC-UV purity 95%. MS m/z 267 (M+1)⁺, calc. 267 (M+1)⁺.

### Step 2: 2-[(2R)-2-Methylpiperazin-1-yl]-3-[2-({2-[2-(1-methylpyrrolidin-2-yl)ethoxy]pyridin-3-yl}oxy)ethoxy]pyrazine, trifluoroacetate.

The procedure in Example 3, Step 2, was followed using 2-({2-[2-(1-methylpyrrolidin-2-yl)ethoxy]pyridin-3-yl}oxy)ethanol (from Step 1; 28 mg, 0.11 mmol) to give 33 mg (0.059 mmol, 59%) of the title compound as a brown oil. LC-UV purity 100%. HRMS m/z calcd for C₂₃H₃₄N₆O₃ (M)⁺ 442.2692, found 442.2681.

### EXAMPLE 5

### 2-[(2R)-2-Methylpiperazin-1-yl]-3-(2-{[2-(piperidin-3-ylmethoxy)pyridin-3-yl]oxy}ethoxy)pyrazine, trifluoroacetate.

### Step 1: 2-{[2-(Piperidin-3-ylmethoxy)pyridin-3-yl]oxy}ethanol.

A solution of 2-chloro-3-[2-(tetrahydro-2*H*-pyran-2-yloxy)ethoxy]pyridine (from Example 1; 100 mg, 0.39 mmol), 3-piperidinemethanol (65 µl, 0.58 mmol) and 1.0 M potassium *tert-*butoxide in *tert*-butanol (0.8 mL, 0.80 mmol) in 4 mL of toluene was heated at 100 °C for 1 day. The organic phase was washed with 3x2 mL of water and 2 mL of brine. The organic phase was shaken at 50°C with 4 mL of 2.0 M acetic acid for 2 days. The aqueous phase was washed with 3 x 3 mL of ethyl acetate, made basic by addition of potassium hydroxide, saturated with sodium chloride and extracted with 3 x 2 mL of ethyl acetate. The organic phase was dried (MgSO₄) and concentrated under reduced pressure to give 52 mg (0.21 mmol, 53%) of the title compound as a colorless oil. LC-UV purity 95%. MS *m*/*z* 253 (M+1)⁺, calc. 253 (M+1)⁺.

### Step 2: 2-[(2R)-2-Methylpiperazin-1-yl]-3-(2-{[2-(piperidin-3-ylmethoxy)pyridin-3-yl]oxy}ethoxy)pyrazine, trifluoroacetate.

The procedure in Example 3, Step 2, was followed using 2-{[2-(piperidin-3-ylmethoxy)pyridin-3-yl]oxy}ethanol (from Step 1; 27 mg, 0.11 mmol) to give 37 mg (0.069 mmol, 69%) of the title compound as a brown oil. LC-UV purity 95%. HRMS *mlz* calcd for C₂₂H₃₂N₆O₃ (M)⁺ 428.2536, found 428.2543.

### EXAMPLE 6

### 2-[(2R)-2-Methylpiperazin-1-yl]-3-{2-[(2-{[(2S)-1-methylpyrrolidin-2-yl]methoxy}pyridin-3-yl)oxy]ethoxy}pyrazine, trifluoroacetate.

### Step 1: 2-[(2-{[(2S)-1-Methylpyrrolidin-2-yl]methoxy}pyridin-3-yl)oxy]ethanol.

A solution of 2-chloro-3-[2-(tetrahydro-2*H*-pyran-2-yloxy)ethoxy]pyridine (from Example 1; 100 mg, 0.39 mmol), (S)-(-)-2-hydroxymethyl-1-methylpyrrolidine (69 µl, 0.58 mmol) and 1.0 M potassium *tert*-butoxide in *tert*-butanol (0.8 mL, 0.80 mmol) in 4 mL of toluene was heated at 100 °C for 1 day. The organic phase was washed with 3 x 2 mL of water and 2 mL of brine. The organic phase was shaken at 50 °C with 4 mL of 2.0 M acetic acid for 2 days. The aqueous phase was washed with 3 x 3 mL of ethyl acetate, made basic by addition of potassium hydroxide, saturated with sodium chloride and extracted with 3 x 2 mL of ethyl acetate. The organic phase was dried (MgSO₄ and concentrated under reduced pressure to give 78 mg (0.31 mmol, 79%) of the title compound as a colorless oil. LC-UV purity 95%. MS m/z 253 (M+1)⁺, calc. 253 (M+1)⁺.

### Step 2: 2-[(2R)-2-Methylpiperazin-1-yl]-3-{2-[(2-{[(2S)-1-methylpyrrolidin-2-yl]methoxy}pyridin-3-yl)oxy]ethoxy}pyrazine trifluoroacetate.

The procedure in Example 3, Step 2, was followed using 2-[(2-{[(2*S*)-1-methylpyrrolidin-2-yl]methoxy}pyridin-3-yl)oxy]ethanol (from Step 1; 37 mg, 0.15 mmol) to give 39 mg (0.071 mmol, 71%) of the title compound as a brown oil. LC-UV purity 95%. HRMS m/z calcd for C₂₂H₃₂N₆O₃ (M)⁺ 428.2536, found 428.2523.

### PREPARATION OF A PHARMACEUTICAL COMPOSITION

### EXAMPLE: Preparation of tablets

| | Ingredients | mg/tablet |
|---|---|---|
| 1. | Active compound of Formula (I) | 10.0 |
| 2. | Cellulose, microcrystalline | 57.0 |
| 3. | Calcium hydrogen phosphate | 15.0 |
| 4. | Sodium starch glycolate | 5.0 |
| 5. | Silicon dioxide, colloidal | 0.25 |
| 6. | Magnesium stearate | 0.75 |

The active ingredient 1 is mixed with ingredients 2, 3, 4 and 5 for about 10 minutes. The magnesium stearate is then added, and the resultant mixture is mixed for about 5 minutes and compressed into tablet form with or without film-coating.

### Pharmacological tests

The ability of a compound of the invention to bind or act at specific 5-HT receptor subtypes can be determined *using in vitro* and *in vivo* assays known in the art. The biological activity of compounds prepared in the Examples was tested using different tests.

### Affinity assays

The receptor affinity of compounds in the Examples was determined in competition experiments, where the ability of each compound in serial dilution to displace ³H-labelled 5-HT, bound to membranes prepared from a transfected HEK293 cell line stably expressing the human 5-HT_{2C} receptor protein, was monitored by Scintillation Proximity Assay technology. Non-specific binding was defined using 5 µM mianserin. Results obtained for exemplary compounds of the invention are illustrated below.

The binding affinities of the compounds for the human 5-HT_{2A} and 5-HT_{2B} receptors in CHO cell lines were determined using ³H-labelled lysergic acid diethyl amide (LSD) and 5-HT, respectively, as radioligands. The binding affinities of the compounds for the human 5-HT_{1A} and 5-HT_{1B} receptors in CHO cell lines were determined similarly using ³H-labelled 8-hydroxy-2-(di-n-propylamino)tetralin (8-OH-DPAT) and 5-HT, respectively, as radioligands.

### In vitro functional assays

The agonist efficacy at the 5-HT_{2C} receptor of the compounds in the Examples was determined by the ability of each compound to mobilise intracellular calcium in transfected HEK293 cells, stably expressing the human 5-HT_{2C} receptor protein, using the calcium-chelating fluorescent dye FLUO-3 (Sigma, St. Louis, MO, U.S.A.).

The ability of the compounds to mobilise intracellular calcium at the 5HT_{2A} and 5-HT_{2B} receptors was determined similarly using CHO cells expressing the human 5-HT_{2A} or human 5-HT_{2B} receptors.

The maximum functional responses of the compounds, at 1 µM, at the 5-HT_{2A}, 5-HT_{2B} and 5-HT_{2C} receptors are expressed relative to the maximum response of 5-HT (serotonin) at a concentration of 1 µM.

## Claims

1. A compound of Formula (I): 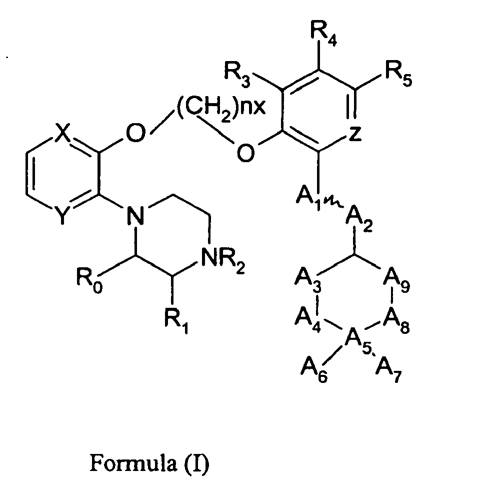 wherein
nx is 2-4, preferably 2;
R₀ and R₁ are each independently H or CH₃;
R₂ is H, C₁-C₄-alkyl, 2-hydroxyethyl, 2-cyanoethyl or tetrahydropyran-2-yl, C₁-C₄-acyl or C₁-C₄-alkoxycarbonyl;
R₃-R₅ are each independently H, halogen, methyl or methoxy, provided that at least one of R₃-R₅ is hydrogen;
X, Y, and Z are each independently CH or N;
A₁ is O, CH or CH₂;
A₂ is O, CH or (CH₂)ₙ₂, wherein n2 is an integer 0-2;
the bond between A₁ and A₂ is a single or double bond;
A₃ is (CH₂)ₙ₃, wherein n3 an integer 0-10, preferably 0-7, more preferably 0-5;
A₄ is (CMe₂)ₙ₄, wherein n4 is an integer 0-1;
A₅ is N or O;
provided that when A₅ is N , then A₅ is substituted by only two of A₆, A₇ and A₈; when A₅ is O, then A₅ is substituted by only one of A₆, A₇ and A₈;
A₆ and A₇ are each independently H, C₁-C₄-alkyl, amino-C₂-C₄-alkyl, N,N-di-C₁-C₄-alkylamino-C₂-C₄-alkyl, C₃-C₆-cycloalkyl or form together with A₅ a saturated heterocyclic ring;
A₈ is (CH₂)ₙ₈, wherein n8 is an integer 0-2;
A₉ is CH₂;
or pharmaceutically acceptable salts, solvates, hydrates, geometrical isomers, tautomers, optical isomers, and N-oxides thereof.

2. The compound according to claim 1, wherein R₁ is hydrogen.

3. The compound according to any one of claims 1 to 2, wherein X and Y both are nitrogen.

4. The compound according to any one of claims 1 to 3, wherein R₂ is H or methyl.

5. The compound according to any one of claims 1 to 4, wherein all of R₃-R₅ are H.

6. The compound according to any one of claims 1 to 5, wherein A₆ and A₇ are each independently H, methyl, isopropyl, 2-ethylamine or form together a pyrrolidine or piperazine ring.

7. The compound according to any one of claims 1 to 6, which has the Formula (Ib): 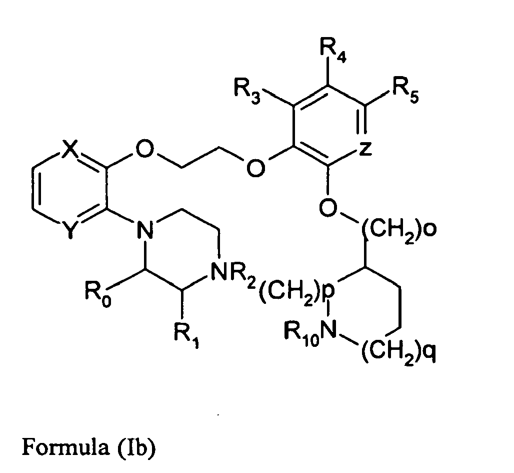 wherein:
R₀-R₅, X, Y, and Z are as in any one of claims 1 to 5,
o is an integer 0-2;
p is an integer 0-2, wherein o and p are preferably not both 0;
q is an integer 0-1;
R₁₀ is H or C₁-C₄-alkyl, preferably H or methyl.

8. The compound according to claim 7, which compound is:
2-[(2R)-2-Methylpiperazin-1-yl]-3-[2-({2-[(1-methylpiperidin-4-yl)oxy]pyridin-3-yl}oxy)ethoxy]pyrazine, trifluoroacetate;
2-[(2R)-2-Methylpiperazin-1-yl]-3-[2-({2-[2-(1-methylpyrrolidin-2-yl)ethoxy]pyridin-3-yl}oxy)ethoxy]pyrazine, trifluoroacetate;
2-[(2R)-2-Methylpiperazin-1-yl]-3-(2-{[2-(piperidin-3-ylmethoxy)pyridin-3-yl]oxy}ethoxy)pyrazine, trifluoroacetate;
2-[(2*R*)-2-Methylpiperazin-1-yl]-3-12-[(2-{[(2*S*)-1-methylpyrrolidin-2-yl]methoxy}pyridin-3-yl)oxy]ethoxy}pyrazine, trifluoroacetate.

9. The compound according to any one of claims 1 to 8 for use in therapy of a human being or an animal.

10. The compound according to claim 9 wherein the therapy is directed towards treatment of a serotonin-related disorder or condition related to the 5-HT_{2C} receptor.

11. The compound according to claim 10, wherein the serotonin-related disorder or condition is selected from memory disorders, such as Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

12. Use of a compound according to any one of claims 1 to 8 for the manufacture of a medicament for treating or preventing a serotonin-related disorder or condition related to the 5-HT_{2C} receptor.

13. The use according to claim 12, wherein the serotonin-related disorder or condition is selected from memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

14. A pharmaceutical composition comprising a compound according to any one of claims 1 to 8 as an active ingredient, together with a pharmaceutically acceptable carrier.

15. A pharmaceutical composition for the treatment of a serotonin-related disorder or condition, particularly 5-HT_{2C} receptor related, comprising administering to a subject in need thereof an effective amount of a compound according to any of claims 1 to 8.

16. A pharmaceutical composition according to claim 15, wherein the serotonin-related disorder or condition is selected from memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

17. A pharmaceutical composition according to any of claims 15 or 16, wherein the subject is a human.

18. A pharmaceutical composition according to any of claims 15 or 16, wherein the subject is an animal.

19. A pharmaceutical composition for modulating 5-HT_{2C} receptor function, comprising contacting the receptor with an effective stimulatory or inhibitory, preferably stimulatory, amount of a compound according to any of claims 1 to 8.

20. A process for the preparation of a compound of the Formula (Ib) according to any one of claims 7 or 8, which process comprises:
a) reacting a compound of Formula (II) 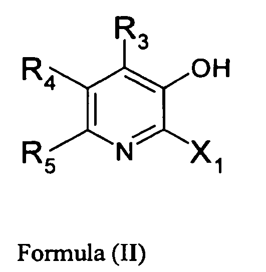 wherein:
X₁ is selected from F, Cl, Br and I,
R₃-R₅ are each independently H, halogen, methyl or methoxy, provided that at least one of R₃-R₅ is hydrogen,
with a compound of Formula (III): 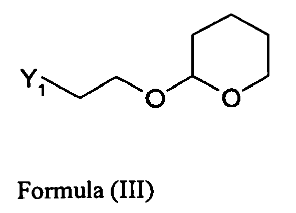 wherein Y₁ is a suitable leaving group selected from Cl, Br, I, OTs, or OMs;
in the presence of a base, such as potassium carbonate, triethylamine, or pyridine, in a solvent, such as acetonitrile, to give a compound of Formula (IV): 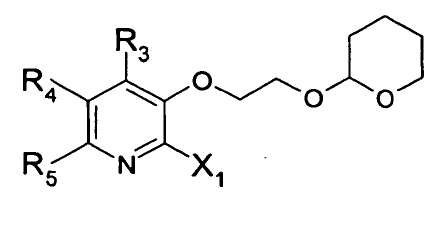 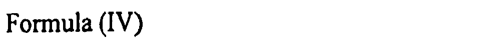 wherein X₁ and R₃-R₅ are as defined above;
b) reacting the compound of Formula (IV) with a compound of Formula (Vb) in the presence of a base, such as potassium tert-butoxide, in a solvent, such as toluene, 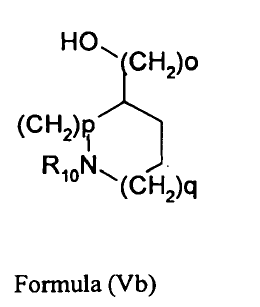 wherein:
o is an integer 0-2;
p is an integer 0-2, wherein o and p are preferably not both 0;
q is an integer 0-1;
R₁₀ is H or C₁-C₄-alkyl, preferably H or methyl;
to give a compound of Formula (VIb): 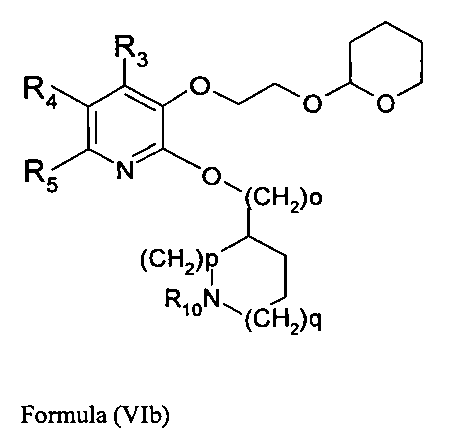 wherein:
R₃-R₅ and R₁₀, o, p, and q are as defined above,
c) treating the compound of Formula (VIb) with an aqueous acid such as aqueous acetic acid or aqueous hydrochloric acid, to give a compound of Formula (VIIb): 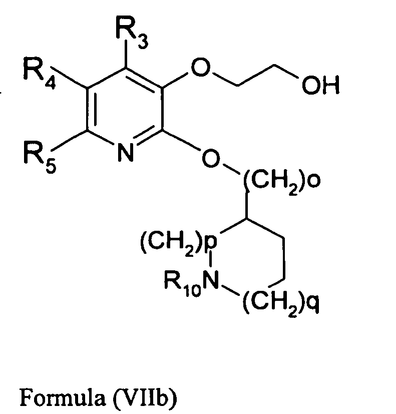 wherein:
R₃-R₅ and R₁₀, o, p, and q are as defined above,
d) reacting the compound of Formula (VIIb) with a compound of Formula (VIII) in the presence of a base such as potassium tert-butoxide, in a solvent, such as methyl tert-butyl ether or toluene, 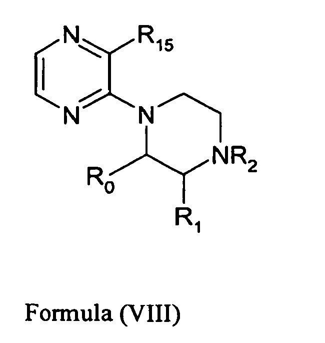 wherein
R₀ and R₁ are each independently H or CH₃;
R₂ is selected from C₁-C₄-alkoxycarbonyl, benzyl, trityl, C₁-C₄-alkyl, 2-hydroxyethyl, 2-cyanoethyl, tetrahydropyran-2-yl, and C₁-C₄-acyl,
R₁₅ is halogen, such as chlorine, to give a compound of Formula (Ib) wherein X = N and Y=N: 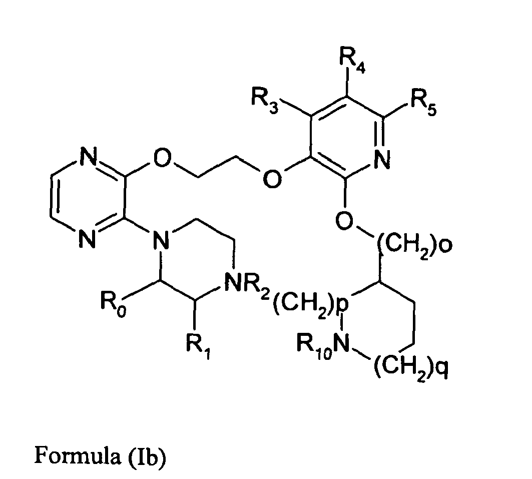 wherein R₀-R₅, R₁₀, o, p, and q are as defined above;
e) if desired, separating a racemate obtained into optical isomers and/or forming an acid addition salt with an organic or inorganic acid,
f) if R₂ in Formula (Ib) following step d) is a nitrogen protecting group, such as t-BOC, trityl, and benzyl, removing said nitrogen protecting group, such as under acidic conditions (e.g. trifluoroacetic acid in a solvent such as chloroform), hydrogenolytic or non-hydrogenolytic conditions, to provide the compound of Formula (Ib), wherein R₂ is hydrogen.

21. A process for the preparation of a compound of the Formula (Ib) according to any one of claims 7 or 8, which process comprises:
a) reacting a compound of Formula (IX) with a benzylating agent, such as benzyl chloride, benzyl bromide, or benzyl tosylate, in the presence of a base: 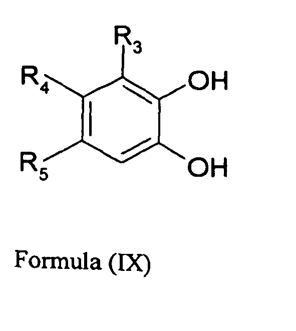 wherein R₃-R₅ are each independently H, halogen, methyl, or methoxy, provided that at least one of R₃-R₅ is hydrogen, to give a compound of Formula (X): 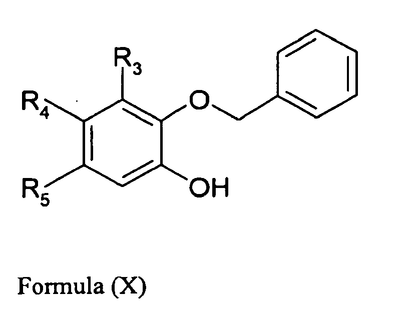 wherein R₃-R₅ are as defined above,
b) reacting the compound of Formula (X) with a compound of Formula (XIb) in the presence of a base, such as potassium carbonate, in a solvent, such as acetone: 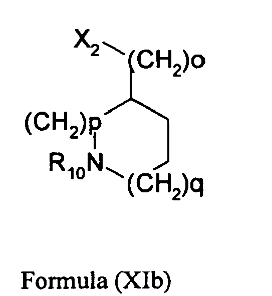 wherein:
X₂ is halogen, OMs, or OTs;
o is an integer 0-2;
p is an integer 0-2, wherein o and p are preferably not both 0;
q is an integer 0-1;
R₁₀ is H or C₁-C₄-alkyl, preferably H or methyl;
to give a compound of Formula (XIIb): 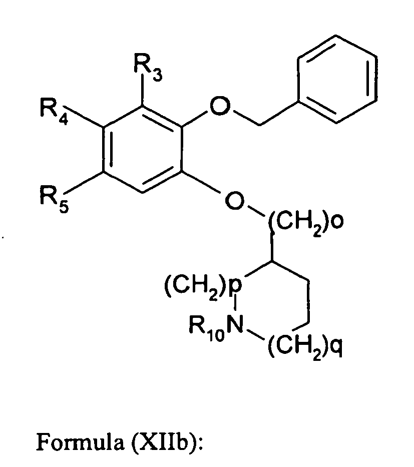 wherein:
R₃-R₅, R₁₀) o, p, and q are as defined above,
c) treating the compound of Formula (XIIb) with hydrogen in the presence of a hydrogenation catalyst, such as palladium on carbon, in a solvent, such as methanol, to give a compound of Formula (XIIIb): 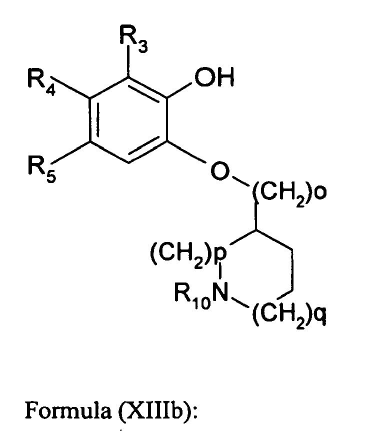 wherein:
R₃-R₅, R₁₀, o, p, and q are as defined above,
d) reacting the compound of Formula (XIIIb) with a hydroxyethylating agent such as ethylene carbonate in the presence of a base, such as potassium carbonate, in a solvent, such as N,N-dimethylformamide, to give a compound of Formula (XIVb): 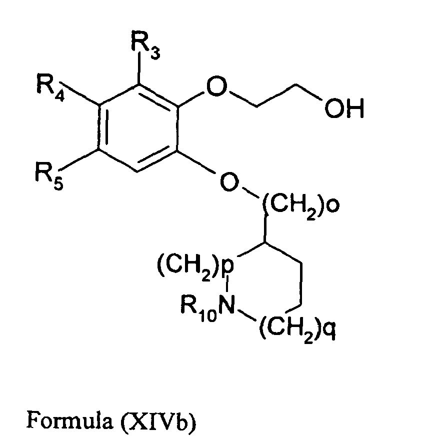 wherein R₃-R₅, R₁₀, o, p, and q are as defined above;
e) reacting the compound of Formula (XIVa) with a compound of Formula (VIII) in the presence of a base, such as potassium tert-butoxide, in a solvent, such as N,N-dimethylformamide: 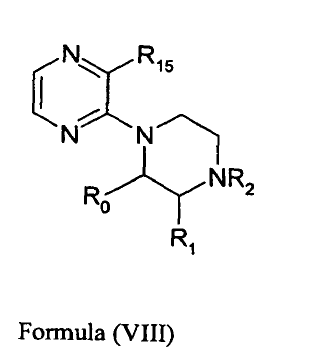 wherein
R₀ and R₁ are each independently H or CH₃;
R₂ is selected from C₁-C₄-alkoxycarbonyl, benzyl, trityl, C₁-C₄-alkyl, 2-hydroxyethyl, 2-cyanoethyl, tetrahydropyran-2-yl, and C₁-C₄-acyl,
R₁₅ is halogen, such as chlorine, to give a compound of Formula (Ib) wherein X = N and Y=N: 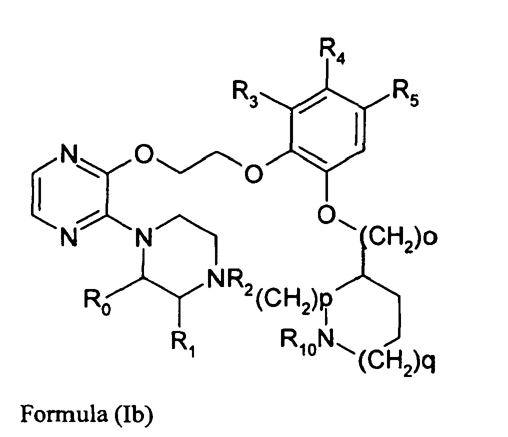 wherein R₀-R₅, R₁₀, o, p, and q are as defined above;
f) if desired, separating a racemate obtained into optical isomers and/or forming an acid addition salt with an organic or inorganic acid,
g) if R₂ in Formula (Ib) following step e) is a nitrogen protecting group, such as t-BOC, trityl, and benzyl, removing said nitrogen protecting group, such as under acidic conditions (e.g. trifluoroacetic acid in a solvent such as chloroform), hydrogenolytic or non-hydrogenolytic conditions, to provide the compound of Formula (Ib), wherein R₂ is hydrogen.

22. A process for the preparation of a compound of the Formula (Ib) according to any one of claims 7 or 8, which process comprises:
a) reacting a compound of Formula (XV) with a compound selected from benzyl chloride, benzyl bromide, benzyl iodide, benzyl tosylate, and benzyl mesylate in the presence of a base, such as potassium carbonate, in a solvent, such as N,N-dimethylformamide, 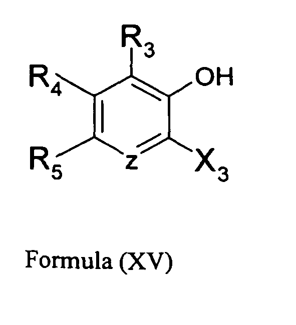 wherein:
X₁ is selected from Cl, Br and I, Z is CH or N,
R₃-R₅ are each independently H, halogen, methyl or methoxy, provided that at least one of R₃-R₅ is hydrogen, to give a compound of Formula (XVI): 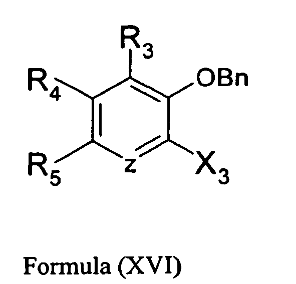 wherein R₃-R₅, X₃, and Z are as defined above;
b) reacting the compound of Formula (XVI) with a compound of Formula (Vb) in the presence of a base, such as sodium tert-butoxide, in a solvent, such as N,N-dimethylformamide, 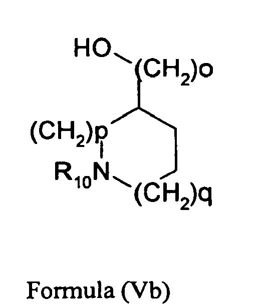 wherein:
o is an integer 0-2;
p is an integer 0-2, wherein o and p are preferably not both 0;
q is an integer 0-1;
R₁₀ is H or C₁-C₄-alkyl, preferably H or methyl;
to give a compound of Formula (XVIIb): 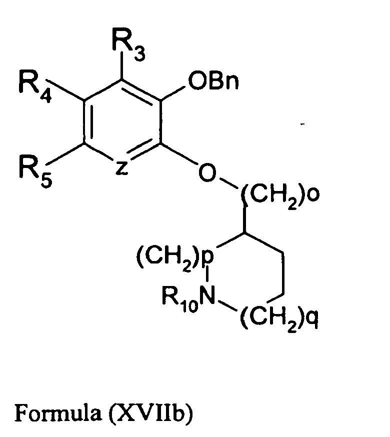 wherein:
R₃-R₅ and R₁₀, o, p, q, and Z are as defined above,
c) treating the compound of Formula (XVIIb) with a hydrogenation catalyst using a suitable hydrogen source such as ammonium formate and then heating in the presence of a hydroxyethylating agent, preferably ethylene carbonate, and a base, such as potassium carbonate, in a solvent, such as N,N-dimethylformamide, to give a compound of Formula (VIIb): 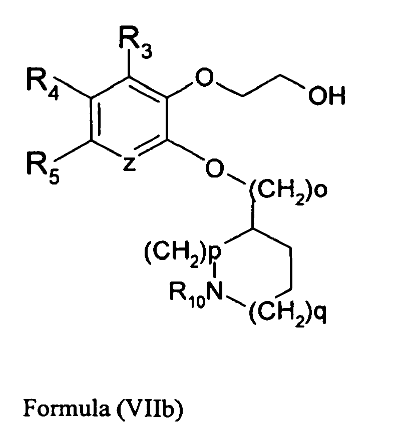 wherein:
R₃-R₅and R₁₀, o, p, q, and Z are as defined above,
d) reacting the compound of Formula (VIIb) with a compound of Formula (VIII) in the presence of a base such as sodium tert-butoxide, in a solvent, such as N,N-dimethylformamide, 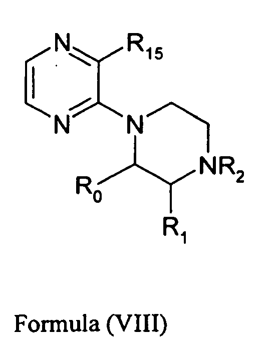 wherein
R₀ and R₁ are each independently H or CH₃;
R₂ is selected from C₁-C₄-alkoxycarbonyl, benzyl, trityl, C₁-C₄-alkyl, 2-hydroxyethyl, 2-cyanoethyl, tetrahydropyran-2-yl, and C₁-C₄-acyl,
R₁₅ is halogen, such as chlorine, to give a compound of Formula (Ib) wherein X = N and Y=N: 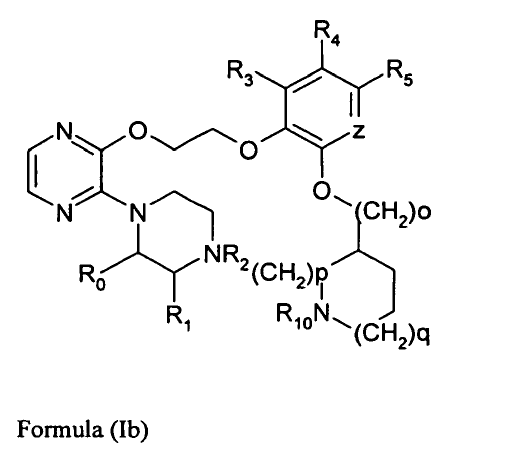
wherein R₀-R₅, R₁₀, o, p, q, and Z are as defined above;
e) if desired, separating a racemate obtained into optical isomers and/or forming an acid addition salt with an organic or inorganic acid,
f) if R₂ in Formula (Ib) following step d) is a nitrogen protecting group, such as *t*-BOC, trityl, and benzyl, removing said nitrogen protecting group, such as under acidic conditions (e.g. trifluoroacetic acid in a solvent such as chloroform), hydrogenolytic or non-hydrogenolytic conditions, to provide the compound of Formula (Ib), wherein R₂ is hydrogen.

23. A pharmaceutical composition for treating a disorder or condition, comprising administering to a subject in need thereof an effective amount of a compound according to any one of claims 1 to 8, wherein the disorder or condition is selected from memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

24. Use of a compound according to any one of claims 1 to 8 for the manufacture of a medicament for treating or preventing memory disorders including Alzheimer's disease; schizophrenia; mood disorders such as depression; anxiety disorders; pain; substance abuse; sexual dysfunctions such as erectile dysfunction; epilepsy; glaucoma; urinary disorders, such as urinary incontinence; menopausal and post-menopausal hot flushes; type 2 diabetes; eating disorders, such as binge eating disorders, anorexia nervosa and bulimia; weight gain associated with antipsychotic drug administration, premenstrual tension, sleep disorders; and particularly obesity.

## Patentansprüche

1. Verbindung der Formel (I): 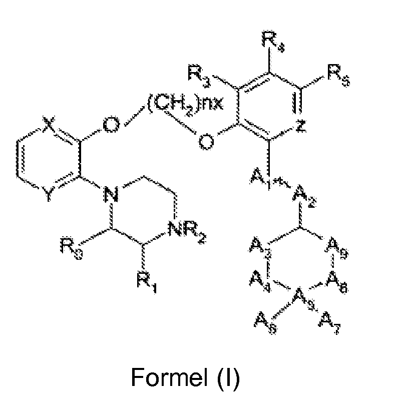 worin
nx 2-4, vorzugsweise 2, ist;
R₀ und R₁ jeweils unabhängig voneinander H oder CH₃ sind;
R₂ H, C₁-C₄-Alkyl, 2-Hydroxyethyl, 2-Cyanethyl oder Tetrahydropyran-2-yl, C₁-C₄-Acyl oder C₁-C₄-Alkoxycarbonyl ist;
R₃-R₅ jeweils unabhängig voneinander H, Halogen, Methyl oder Methoxy sind, mit der Maßgabe, dass von R₃-R₅ zumindest eines Wasserstoff ist;
X, Y und Z jeweils unabhängig voneinander CH oder N sind;
A₁ O, CH oder CH₂ ist;
A₂O, CH oder (CH₂)ₙ₂ ist, worin n2 eine ganze Zahl von 0-2 ist;
die Bindung zwischen A₁ und A₂ eine Einfach- oder Doppelbindung ist;
A₃ (CH₂)ₙ₃ ist, worin n3 eine ganze Zahl von 0-10, vorzugsweise von 0-7, besonders bevorzugt von 0-5, ist;
A₄ (CMe₂)n₄ ist, worin n4 eine ganze Zahl von 0-1 ist;
A₅ N oder O ist;
mit der Maßgabe, dass, wenn A₅ N ist, A₅ dann nur durch zwei von A₆, A₇ und A₈ substituiert ist; wenn A₅ O ist, A₅ dann nur durch eines von A₆, A₇ und A₈ substituiert ist;
A₆ und A₇ jeweils unabhängig voneinander H, C₁-C₄-Alkyl, Amino-C₂-C₄-alkyl, N,N-Di-C₁-C₄-alkylamino-C₂-C₄-alkyl, C₃-C₆-Cycloalkyl sind oder zusammen mit A₅ einen gesättigten heterozyklischen Ring bilden;
A₈ (CH₂)ₙ₈ ist, worin n8 eine ganze Zahl von 0-2 ist;
A₉ CH₂ ist;
oder pharmazeutisch annehmbare Salze, Solvate, Hydrate, geometrische Isomere, Tautomere, optische Isomere und N-Oxide davon.

2. Verbindung nach Anspruch 1, worin R₁ Wasserstoff ist.

3. Verbindung nach einem der Ansprüche 1 bis 2, worin X und Y beide Stickstoff sind.

4. Verbindung nach einem der Ansprüche 1 bis 3, worin R₂ H oder Methyl ist.

5. Verbindung nach einem der Ansprüche 1 bis 4, worin R₃-R₅ alle H sind.

6. Verbindung nach einem der Ansprüche 1 bis 5, worin A₆ und A₇ jeweils unabhängig voneinander H, Methyl, Isopropyl, 2-Ethylamin sind oder zusammen einen Pyrrolidin- oder Piperazinring bilden.

7. Verbindung nach einem der Ansprüche 1 bis 6, umfassend die Formel (Ib): 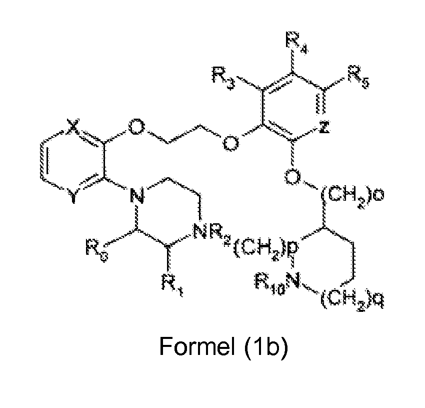 worin:
R₀-R₅, X, Y und Z wie in einem der Ansprüche 1 bis 5 sind,
o eine ganze Zahl von 0-2 ist;
p eine ganze Zahl von 0-2 ist, worin o und p vorzugsweise nicht beide 0 sind;
q eine ganze Zahl von 0-1 ist;
R₁₀ H oder C₁-C₄-Alkyl, vorzugsweise H oder Methyl, ist.

8. Verbindung nach Anspruch 7, wobei die Verbindung ist:
2-[(2*R*)-2-Methylpiperazin-1-yl]-3-[2-({2-[(1-methylpiperidin-4-yl)oxy]pyridin-3-yl}oxy)ethoxy]pyrazin-trifluoracetat;
2-[(2*R*)-2-Methylpiperazin-1-yl]-3-[2-({2-[2-(1-methylpyrrolidin-2-yl)ethoxy]pyridin-3-yl}oxy)ethoxy]pyrazin-trifluoracetat;
2-[(2*R*)-2-Methylpiperazin-1-yl]-3-(2-{[2-(piperidin-3-ylmethoxy)pyridin-3-yl]oxy}ethoxy)pyrazin-trifluoracetat;
2-[(2*R*)-2-Methylpiperazin-1-yl]-3-{2-[(2-{[(2*S*)-1-methylpyrrolidin-2-yl]methoxy}pyridin-3-yl)oxy]ethoxy}pyrazin-trifluoracetat.

9. Verbindung nach einem der Ansprüche 1 bis 8 zur Verwendung in der Therapie eines Menschen oder eines Tiers.

10. Verbindung nach Anspruch 9, wobei die Therapie auf die Behandlung einer mit Serotonin in Zusammenhang stehenden Störung oder Erkrankung, die in Verbindung mit dem 5-HT_{2C}-Rezeptor steht, gerichtet ist.

11. Verbindung nach Anspruch 10, wobei die mit Serotonin in Zusammenhang stehende Störung oder Erkrankung aus Gedächtsnisstörungen, wie beispielsweise Alzheimer-Krankheit; Schizophrenie; Gemütsstörungen, wie beispielsweise Depression; Angststörungen; Schmerz, Suchtmittelmissbrauch; sexuellen Dysfunktionen, wie beispielsweise erektiler Dysfunktion; Epilepsie; Glaukom; Harnbeschwerden, wie beispielsweise Harninkontinenz; Hitzewallungen im Klimakterium und nach dem Klimakterium; Diabetes vom Typ 2; Essstörungen, wie beispielsweise Essstörungen mit Fressattacken, Magersucht und Bulimie; Gewichtszunahme in Zusammenhang mit einer antipsychotischen Medikamentenverabreichung, prämenstrueller Spannung, Schlafstörungen; und insbesondere Fettleibigkeit ausgewählt ist.

12. Verwendung einer Verbindung nach einem der Ansprüche 1 bis 8 zur Herstellung eines Medikaments zur Behandlung oder Verhinderung einer mit Serotonin in Zusammenhang stehenden Störung oder Erkrankung, die mit dem 5-HT_{2C}-Rezeptor in Verbindung steht.

13. Verwendung nach Anspruch 12, wobei die mit Serotonin in Zusammenhang stehende Störung oder Erkrankung aus Gedächtnisstörungen einschließlich Alzheimer-Erkrankung; Schizophrenie; Gemütsstörungen, wie beispielsweise Depression; Angststörungen; Schmerz; Suchtmittelmissbrauch; sexuellen Dysfunktionen, wie beispielsweise erektiler Dysfunktion; Epilepsie; Glaukom; Harnbeschwerden, wie beispielsweise Harninkontinenz; Hitzewallungen im Klimakterium und nach dem Klimakterium; Diabetes vom Typ 2; Essstörungen, wie beispielsweise Essstörungen mit Fressattacken, Magersucht und Bulimie; Gewichtszunahme in Zusammenhang mit einer antipsychotischen Medikamentenverabreichung, prämenstrueller Spannung, Schlafstörungen; und insbesondere Fettleibigkeit ausgewählt ist.

14. Pharmazeutische Zusammensetzung, umfassend eine Verbindung nach einem der Ansprüche 1 bis 8 als Wirkstoff zusammen mit einem pharmazeutisch annehmbaren Träger.

15. Pharmazeutische Zusammensetzung zur Behandlung einer mit Serotonin in Zusammenhang stehenden Störung oder Erkrankung, die insbesondere mit dem 5-HT_{2C}-Rezeptor in Verbindung steht, umfassend die Verabreichung einer wirksamen Menge einer Verbindung nach einem der Ansprüche 1 bis 8 an ein diese benötigendes Lebewesen.

16. Pharmazeutische Zusammensetzung nach Anspruch 15, wobei die mit Serotonin in Zusammenhang stehende Störung oder Erkrankung aus Gedächtnisstörungen, einschließlich Alzheimer-Krankheit, Schizophrenie; Gemütsstörungen, wie beispielsweise Depression; Angststörungen; Schmerz; Suchtmittelmissbrauch; sexuellen Dysfunktionen, wie beispielsweise erektiler Dysfunktion; Epilepsie; Glaukom; Harnbeschwerden, wie beispielsweise Harninkontinenz; Hitzewallungen im Klimakterium und nach dem Klimakterium; Diabetes vom Typ 2; Essstörungen, wie beispielsweise Essstörungen mit Fressattacken, Magersucht und Bulimie; Gewichtszunahme in Zusammenhang mit einer antipsychotischen Medikamentenverabreichung, prämenstrueller Spannung, Schlafstörungen; und insbesondere Fettleibigkeit ausgewählt ist.

17. Pharmazeutische Zusammensetzung nach einem der Ansprüche 15 oder 16, wobei das Lebewesen ein Mensch ist.

18. Pharmazeutische Zusammensetzung nach einem der Ansprüche 15 oder 16, wobei das Lebewesen ein Tier ist.

19. Pharmazeutische Zusammensetzung zur Modulation der 5-HT_{2C}-Rezeptorfunktion, umfassend das Kontaktieren des Rezeptors mit einer wirksamen stimulatorischen oder inhibitorischen, vorzugsweise stimulatorischen, Menge einer Verbindung nach einem der Ansprüche 1 bis 8.

20. Verfahren zur Herstellung einer Verbindung der Formel (Ib) nach einem der Ansprüche 7 oder 8, wobei das Verfahren umfasst:
a) das Reagieren einer Verbindung der Formel (II) 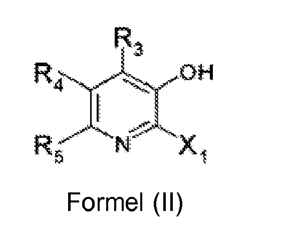 worin:
X₁ aus F, Cl, Br und I ausgewählt ist,
R₃-R₅ jeweils unabhängig voneinander H, Halogen, Methyl oder Methoxy sind, mit der Maßgabe, dass von R₃-R₅ zumindest eines Wasserstoff ist,
mit einer Verbindung der Formel (III): 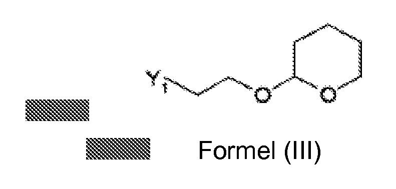 worin Y₁ eine geeignete Abgangsgruppe ist, die aus Cl, Br, I, OTs oder OMs ausgewählt ist;
in Gegenwart einer Base, wie beispielsweise Kaliumcarbonat, Triethylamin oder Pyridin, in einem Lösungsmittel, wie beispielsweise Acetonitril, zu einer Verbindung der Formel (IV): 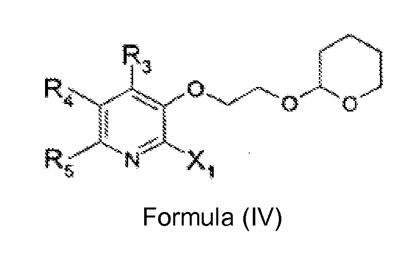 worin X₁ und R₃-R₅ wie oben definiert sind;
b) das Reagieren der Verbindung der Formel (IV) mit einer Verbindung der Formel (Vb) in Gegenwart einer Base, wie beispielsweise Kalium-tert.-butoxid, in einem Lösungsmittel, wie beispielsweise Toluol, 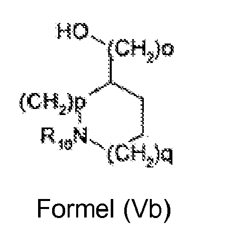 worin:
o eine ganze Zahl von 0-2 ist;
p eine ganze Zahl von 0-2 ist, worin o und p vorzugsweise nicht beide 0 sind;
q eine ganze Zahl von 0-1 ist;
R₁₀ H oder C₁-C₄-Alkyl, vorzugsweise H oder Methyl, ist;
zu einer Verbindung der Formel (VIb): 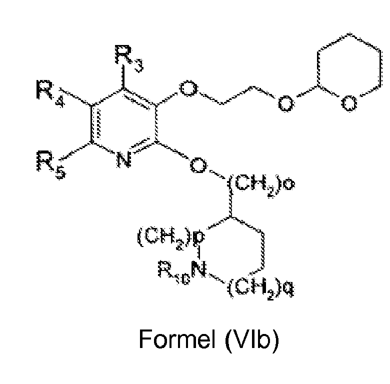 worin:
R₃-R₅ und R₁₀, o, p und q wie oben definiert sind,
c) das Behandeln der Verbindung der Formel (VIb) mit einer wässrigen Säure, wie beispielsweise wässriger Essigsäure oder wässriger Chlorwasserstoffsäure, um eine Verbindung der Formel (VIIb) zu ergeben: 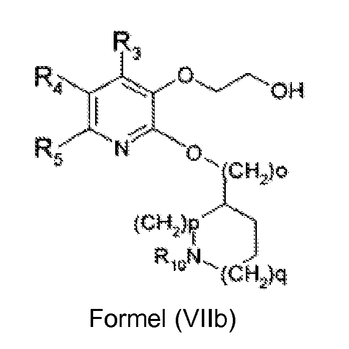 worin:
R₃-R₅ und R₁₀, o, p und q wie oben definiert sind,
d) das Reagieren der Verbindung der Formel (VIIb) mit einer Verbindung der Formel (VIII) in Gegenwart einer Base, wie beispielsweise Kalium-tert.-butoxid, in einem Lösungsmittel, wie beispielsweise Methyl-tert.-butylether oder Toluol, 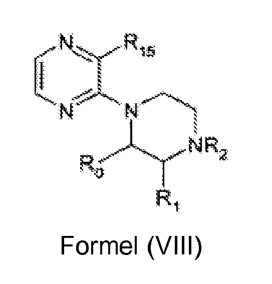 worin
R₀ und R₁ jeweils unabhängig voneinander H oder CH₃ sind;
R₂ aus C₁-C₄-Alkoxycarbonyl, Benzyl, Trityl, C₁-C₄-Alkyl, 2-Hydroxyethyl, 2-Cyanethyl, Tetrahydropyran-2-yl und C₁-C₄-Acyl ausgewählt ist,
R₁₅ Halogen, wie beispielsweise Clor, ist, zu einer Verbindung der Formel (Ib), worin X=N und Y=N: 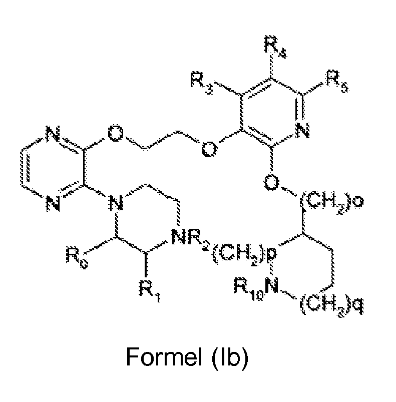 worin R₀-R₅, R₁₀, o, p und q wie oben definiert sind;
e) falls gewünscht, das Auftrennen eines erhaltenen Razemats in optische Isomere und/oder das Bilden eines Säureadditionssalzes mit einer organischen oder anorganischen Säure,
f) wenn R₂ in der Formel (Ib) im Anschluss an den Schritt d) eine Stickstoffschutzgruppe, wie beispielsweise t-BOC, Trityl und Benzyl, ist, das Entfernen der Stickstoffschutzgruppe, wie beispielsweise unter sauren Bedingungen (z.B. Trifluoressigsäure in einem Lösungsmittel, wie beispielsweise Chloroform), hydrogenolytischen oder nicht hydrogenolytischen Bedingungen, um eine Verbindung der Formel (Ib) vorzusehen, worin R₂ Wasserstoff ist.

21. Verfahren zur Herstellung einer Verbindung der Formel (Ib) nach einem der Ansprüche 7 oder 8, wobei das Verfahren umfasst:
a) das Reagieren einer Verbindung der Formel (IX) mit einem Benzylierungsmittel, wie beispielsweise Benzylchlorid, Benzylbromid oder Benzyltosylat, in Gegenwart einer Base: 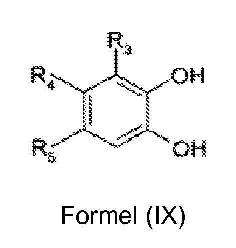 worin R₃-R₅ jeweils unabhängig voneinander H, Halogen, Methyl oder Methoxy sind, mit der Maßgabe, dass von R₃-R₅ zumindest eines Wasserstoff ist, zu einer Verbindung der Formel (X): 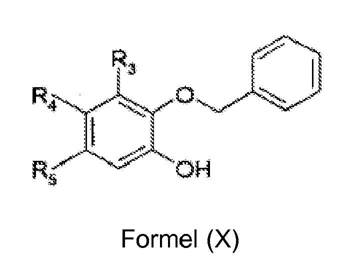 worin R₃-R₅ wie oben definiert sind,
b) das Reagieren der Verbindung der Formel (X) mit einer Verbindung der Formel (XIb) in Gegenwart einer Base, wie beispielsweise Kaliumkarbonat, in einem Lösungsmittel, wie beispielsweise Aceton: 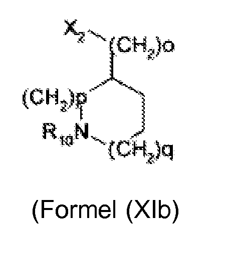 worin:
X₂ Halogen, OMs oder OTs ist;
o eine ganze Zahl von 0-2 ist;
p eine ganze Zahl von 0-2 ist, worin o und p vorzugsweise nicht beide 0 sind;
q eine ganze Zahl von 0-1 ist;
R₁₀ H oder C₁-C₄-Alkyl, vorzugsweise H oder Methyl, ist;
zu einer Verbindung der Formel (XIIb): 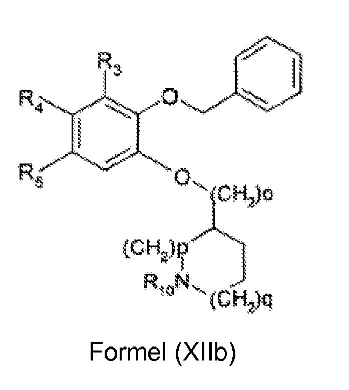 worin:
R₃-R₅, R₁₀, o, p und q wie oben definiert sind,
c) das Behandeln der Verbindung der Formel (XIIb) mit Wasserstoff in Gegenwart eines Hydrierungskatalysators, wie beispielsweise Palladium auf Kohlenstoff, in einem Lösungsmittel, wie beispielsweise Methanol, um eine Verbindung der Formel (XIIIb) zu ergeben: 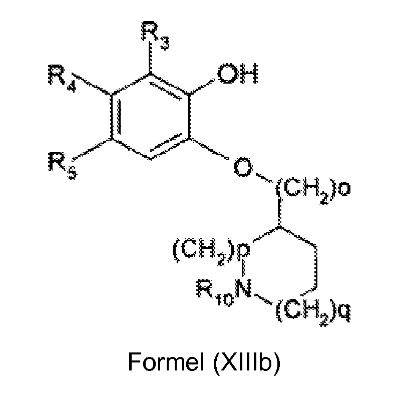 worin:
R₃-R₅, R₁₀, o, p und q wie oben definiert sind,
d) das Reagieren der Verbindung der Formel (XIIIb) mit einem Hydroxyethylierungsmittel, wie beispielsweise Ethylencarbonat, in Gegenwart einer Base, wie beispielsweise Kaliumcarbonat, in einem Lösungsmittel, wie beispielsweise N,N-Dimethylformamid, zu einer Verbindung der Formel (XIVb): 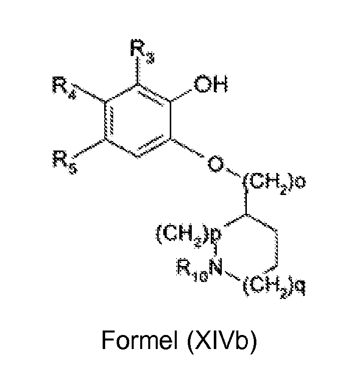 worin R₃-R₅, R₁₀, o, p und q wie oben definiert sind;
e) das Reagieren der Verbindung der Formel (XIVa) mit einer Verbindung der Formel (VIII) in Gegenwart einer Base, wie beispielsweise Kalium-tert.-butoxid, in einem Lösungsmittel, wie beispielsweise N,N-Dimethylformamid: 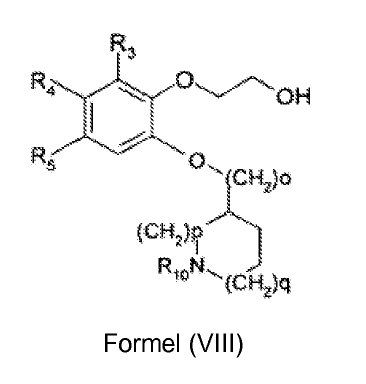 worin
R₀ und R₁ jeweils unabhängig voneinander H oder CH₃ sind;
R₂ aus C₁-C₄-Alkoxycarbonyl, Benzyl, Trityl, C₁-C₄-Alkyl, 2-Hydroxyethyl, 2-Cyanethyl, Tetrahydropyran-2-yl und C₁-C₄-Acyl ausgewählt ist,
R₁₅ Halogen, wie beispielsweise Chlor, ist, zu einer Verbindung der Formel (Ib), worin X=N und Y=N: 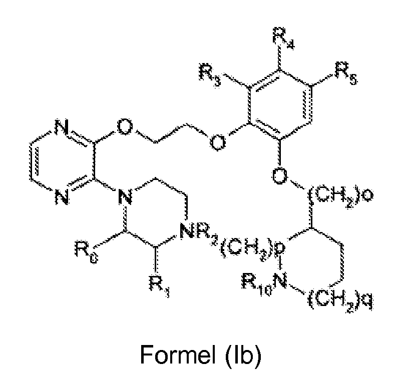
worin R₀-R₅, R₁₀, o, p und q wie oben definiert sind;
f) falls gewünscht, das Auftrennen eines erhaltenen Racemats in optische Isomere und/oder das Bilden eines Säureadditionssalzes mit einer organischen oder anorganischen Säure,
g) wenn R₂ in der Formel (Ib) im Anschluss an den Schritt e) eine Stickstoffschutzgruppe, wie beispielsweise t-BOC, Trityl und Benzyl, ist, das Entfernen der Stickstoffschutzgruppe, wie beispielsweise unter sauren Bedingungen (z.B. Tritfluoressigsäure in einem Lösungsmittel, wie beispielsweise Chloroform), hydrogenolytischen oder nicht hydrogenolytischen Bedingungen, um die Verbindung der Formel (Ib) vorzusehen, worin R₂ Wasserstoff ist.

22. Verfahren zur Herstellung einer Verbindung der Formel (Ib) nach einem der Ansprüche 7 oder 8, wobei das Verfahren umfasst:
a) das Reagieren der Verbindung der Formel (XV) mit einer Verbindung, die aus Benzylchlorid, Benzylbromid, Benzyliodid, Benzyltosylat und Benzylmesylat ausgewählt ist, in Gegenwart einer Base, wie beispielsweise Kaliumcarbonat, in einem Lösungsmittel, wie beispielsweise N,N-Dimethylformamid, 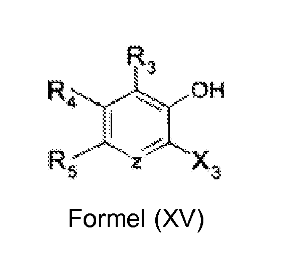 worin:
X₁ aus Cl, Br und I ausgewählt ist, Z CH oder N ist,
R₃-R₅ jeweils unabhängig voneinander H, Halogen, Methyl oder Methoxy sind, mit der Maßgabe, dass von R₃-R₅ zumindest eines Wasserstoff ist, zu einer Verbindung der Formel (XVI): 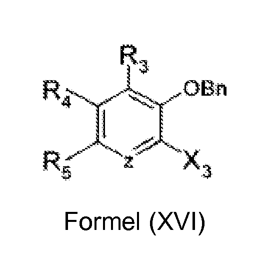 worin R₃-R₅, X₃ und Z wie oben definiert sind;
b) das Reagieren der Verbindung der Formel (XVI) mit einer Verbindung der Formel (Vb) in Gegenwart einer Base, wie beispielsweise Natrium-tert.-butoxid, in einem Lösungsmittel, wie beispielsweise N,N-Dimethylformamid, 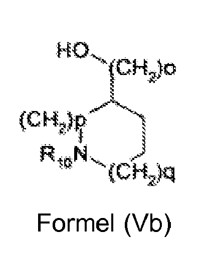 worin:
o eine ganze Zahl von 0-2 ist;
p eine ganze Zahl von 0-2 ist, worin o und p vorzugsweise nicht beide 0 sind;
q eine ganze Zahl von 0-1 ist;
R₁₀ H oder C₁-C₄-Alkyl, vorzugsweise H oder Methyl, ist;
zu einer Verbindung der Formel (XVIIb): 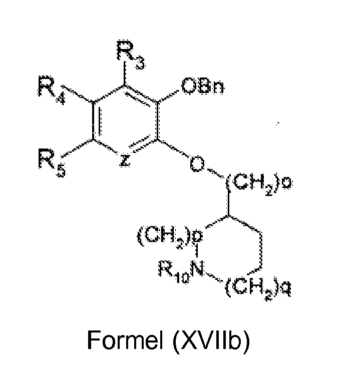 worin:
R₃-R₅ und R₁₀, o, p, q und Z wie oben definiert sind,
c) das Behandeln der Verbindung der Formel (XVIIb) mit einem Hydrierungskatalysator mittels einer geeigneten Wasserstoffquelle, wie beispielsweise Ammoniumformiat, und dann das Erwärmen in Gegenwart eines Hydroxyethylierungsmittels, vorzugsweise Ethylencarbonat, und einer Base, wie beispielsweise Kaliumcarbonat, in einem Lösungsmittel, wie beispielsweise N,N-Dimethylformamid, um eine Verbindung der Formel (VIIb) zu ergeben: 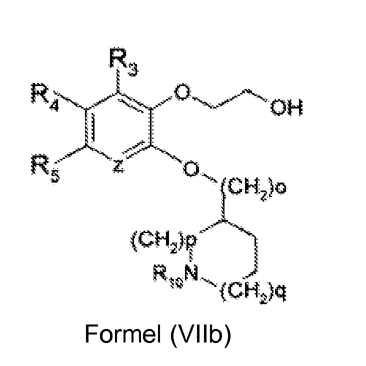 worin:
R₃-R₅ und R₁₀, o, p, q und Z wie oben definiert sind,
d) das Reagieren der Verbindung der Formel (VIIb) mit einer Verbindung der Formel (VIII) in Gegenwart einer Base, wie beispielsweise Natrium-tert.-butoxid, in einem Lösungsmittel, wie beispielsweise N,N-Dimethylformamid, 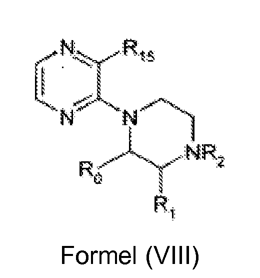 worin
R₀ und R₁ jeweils unabhängig voneinander H oder CH₃ sind;
R₂ aus C₁-C₄-Alkoxycarbonyl, Benzyl, Trityl, C₁-C₄-Alkyl, 2-Hydroxyethyl, 2-Cyanethyl, Tetrahydropyran-2-yl und C₁-C₄-Acyl ausgewählt ist,
R₁₅ Halogen, wie beispielsweise Chlor, ist, zu einer Verbindung der Formel (Ib), worin X=N und Y=N: 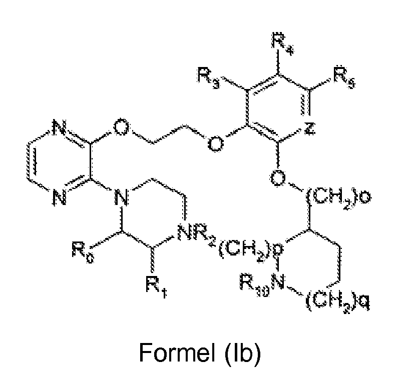 worin R₀-R₅, R₁₀, o, p, q und Z wie oben definiert sind;
e) falls gewünscht, das Auftrennen eines erhaltenen Razemats in optische Isomere und/oder das Bilden eines Säureadditionssalzes mit einer organischen oder anorganischen Säure,
f) wenn R₂ in der Formel (Ib) im Anschluss an den Schritt d) eine Stickstoffschutzgruppe, wie beispielsweise t-BOC, Trityl und Benyzl, ist, das Entfernen der Stickstoffschutzgruppe, wie beispielsweise unter sauren Bedingungen (z.B. Trifluoressigsäure in einem Lösungsmittel, wie beispielsweise Choroform), hydrogenolytischen oder nicht hydrogenolytischen Bedingungen, um die Verbindung der Formel (Ib) vorzusehen, worin R₂ Wasserstoff ist.

23. Pharmazeutische Zusammensetzung zur Behandlung einer Störung oder Erkrankung, umfassend das Verabreichen einer wirksamen Menge einer Verbindung nach einem der Ansprüche 1 bis 8 an ein diese benötigendes Lebewesen, worin die Störung oder die Erkrankung aus Gedächtnisstörungen, einschließlich Alzheimer-Krankheit; Schizophrenie; Gemütsstörungen, wie beispielsweise Depression; Angststörungen; Schmerz; Suchtmittelmissbrauch; sexuellen Dysfunktionen, wie beispilsweise erektiler Dysfunktion; Epilepsie; Glaukom; Harnbeschwerden, wie beispielsweise Harninkontinenz; Hitzewallungen im Klimakterium und nach dem Klimakterium; Diabetes vom Typ 2; Essstörungen, wie beispielsweise Essstörungen mit Fressattacken, Magersucht und Bulimie; Gewichtszunahme in Zusammenhang mit einer antipsychotischen Medikamentenverabreichung, prämenstrueller Spannung, Schlafstörungen; und insbesondere Fettleibigkeit ausgewählt ist.

24. Verwendung einer Verbindung nach einem der Ansprüche 1 bis 8 zur Herstellung eines Medikaments zur Behandlung oder Verhinderung von Gedächtsnisstörungen, einschließlich Alzheimer-Krankheit; Schizophrenie; Gemütsstörungen, wie beispielsweise Depression; Angststörungen; Schmerz; Suchtmittelmissbrauch; sexuellen Dysfunktionen, wie beispielsweise erektiler Dysfunktion; Epilepsie; Glaukom; Harnbeschwerden, wie beispielsweise Harninkontinenz; Hitzewallungen im Klimakterium und nach dem Klimakterium; Diabetes vom Typ 2; Essstörungen, wie beispielsweise Essstörungen mit Fressattacken, Magersucht und Bulimie; Gewichtszunahme in Verbindung mit antipsychotischer Medikamentenverabreichung, prämenstrueller Spannung, Schlafstörungen; und insbesondere Fettsucht.

## Revendications

1. Composé de formule (I) 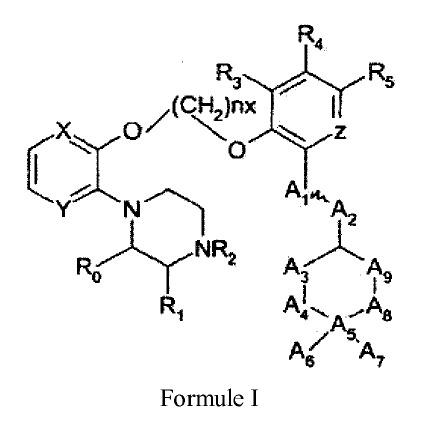 dans laquelle
nx est 2-4, de préférence 2;
R₀ et R₁ sont chacun indépendamment H ou CH₃;
R₂ est H, alkyle en C₁-C₄, 2-hydroxyéthyle, 2-cyanoéthyle ou tétrahydropyran-2- yle, acyle en C₁-C₄ ou (alcoxy en C₁-C₄)carbonyle;
R₃-R₅ sont chacun indépendamment H, halogène, méthyle ou méthoxy, à condition qu'au moins l'un des R₃-R₅ soit l'hydrogène;
X, Y et Z sont chacun indépendamment CH ou N;
A₁ est O, CH ou CH₂;
A₂ est O, CH ou (CH₂)ₙ₂, où n₂ est un entier de 0 à 2; la liaison entre A₁ et A₂ est une liaison simple ou double;
A₃ est (CH₂)ₙ₃ où n3 est un entier de 0 à 10, de préférence de 0 à 7, de façon plus préférable de 0 à 5;
A₄ est (CMe₂)n₄ où n4 est un entier de 0 à 1 ;
A₅ est N ou O; à condition que, lorsque A₅ est N, A₅ ne soit substitué que par deux des A₆, A₇ et A₈, et que, lorsque A₅ est O, A₅ ne soit substitué que par un des A₆, A₇ et A₈,
A₆ et A₇ sont chacun indépendamment H, alkyle en C₁-C₄, aminoalkyle en C₂-C₄, N,N-di(alkyl en C₁-C₄)amino(alkyle en C₂-C₄), cycloalkyle en C₃-C₆, ou forment ensemble avec A₅ un cycle hétérocyclique saturé;
A₈ est (CH₂),₈, où n8 est un entier de 0 à 2;
A₉ est CH₂;
ou ses sels, solvates, hydrates, isomères géométriques, tautomères, isomères optiques et N-oxydes pharmaceutiquement acceptables.

2. Composé selon la revendication 1, dans lequel R₁ est l'hydrogène.

3. Composé selon l'une quelconque des revendications 1 à 2, dans lequel X et Y sont tous les deux l'azote.

4. Composé selon l'une quelconque des revendications 1 à 3, dans lequel R₂ est H ou méthyle.

5. Composé selon l'une quelconque des revendications 1 à 4, dans lequel les R₃-R₅ sont tous H.

6. Composé selon l'une quelconque des revendications 1 à 5, dans lequel A₆ et A₇ sont chacun indépendamment H, méthyle, isopropyle, 2-éthylamine, ou forment ensemble un cycle pyrrolidine ou pipérazine.

7. Composé selon l'une quelconque des revendications 1 à 6, qui a la formule (Ib): 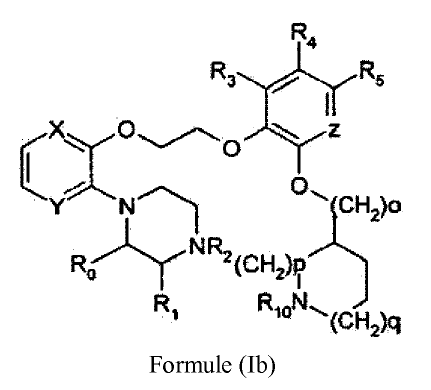 dans laquelle
R₀-R₅, X, Y et Z sont comme dans l'une quelconque des revendications 1 à 5,
o est un entier de 0 à 2;
p est un entier de 0 à 2, o et p n'étant de préférence pas 0 tous les deux;
q est un entier de 0 à 1 ;
R₁₀ est H ou alkyle en C₁-C₄, de préférence H ou méthyle.

8. Composé selon la revendication 7, ce composé étant:
le trifluoroacétate de 2-[(2R)-2-méthylpipérazin-1-yl]-3-[2-({2-[(1-méthylpipéridin-4-yl)oxy]pyridin-3-yl}oxy)éthoxy]pyrazine;
le trifluoroacétate de 2-[(2R)-2-méthylpipérazin-1-yl]-3-[2-((2-[2-(1-méthylpyrrolidin-2-yl)éthoxy]pyridin-3-yl}oxy)éthoxy]pyrazine;
le trifluoroacétate de 2-[(2R)-2-méthylpipérazin-1-yl]-3-(2- {[2-(pipéridin-3-ylméthoxy)pyridin-3-yl]oxy} éthoxy)pyrazine;
le trifluoroacétate de 2-[(2R)-2-méthylpipérazin-1-yl]-3- {2-[(2-{[(2S)-1-méthylpyrrolidin-2-yl]méthoxy}pyridin-3-yl)oxy]éthoxy}pyrazine.

9. Composé selon l'une quelconque des revendications 1 à 8, pour utilisation dans une thérapie d'un être humain ou d'un animal.

10. Composé selon la revendication 9, la thérapie étant dirigée vers le traitement d'un trouble ou d'un état associé à la sérotonine, en relation avec le récepteur 5-HT_{2C}.

11. Composé selon la revendication 10, le trouble ou l'état associé à la sérotonine étant choisi parmi des troubles de la mémoire, comme la maladie d'Alzheimer; la schizophrénie; des troubles de l'humeur comme la dépression; des troubles d'anxiété; la douleur; la toxicomanie; des dysfonctionnements sexuels comme un dysfonctionnement érectile; l'épilepsie; le glaucome; des troubles urinaires comme l'incontinence urinaire; les bouffées de chaleur ménopausiques et post-ménopausiques; le diabète de type 2; des troubles de l'alimentation comme les troubles d'alimentation compulsive (binge eating), l'anorexie nerveuse et la boulimie; la prise de poids associée à l'administration de médicaments antipsychotiques, le syndrome de tension prémenstruelle, les troubles du sommeil; et en particulier l'obésité.

12. Utilisation d'un composé selon l'une quelconque des revendications 1 à 8 pour la fabrication d'un médicament destiné au traitement ou à la prévention d'un trouble ou d'un état associé à la sérotonine, en relation avec le récepteur 5-HT_{2C}.

13. Utilisation selon la revendication 12, dans laquelle le trouble ou l'état associé à la sérotonine est choisi parmi des troubles de la mémoire comprenant la maladie d'Alzheimer; la schizophrénie; des troubles de l'humeur comme la dépression; des troubles d'anxiété; la douleur; la toxicomanie; des dysfonctionnements sexuels comme un dysfonctionnement érectile; l'épilepsie; le glaucome; des troubles urinaires comme l'incontinence urinaire; les bouffées de chaleur ménopausiques et post-ménopausiques; le diabète de type 2; des troubles de l'alimentation comme les troubles d'alimentation compulsive (binge eating), l'anorexie nerveuse et la boulimie; la prise de poids associée à l'administration de médicaments antipsychotiques, le syndrome de tension prémenstruelle, les troubles du sommeil; et en particulier l'obésité.

14. Composition pharmaceutique comprenant un composé selon l'une quelconque des revendications 1 à 8 comme ingrédient actif avec un support pharmaceutiquement acceptable.

15. Composition pharmaceutique pour le traitement d'un trouble ou d'un état associé à la sérotonine, en particulier en relation avec le récepteur 5-HT_{2C}, comprenant l'administration à un sujet en ayant besoin d'une quantité efficace d'un composé selon l'une quelconque des revendications 1 à 8.

16. Composition pharmaceutique selon la revendication 15, le trouble ou l'état associé à la sérotonine étant choisi parmi des troubles de la mémoire comprenant la maladie d'Alzheimer; la schizophrénie; des troubles de l'humeur comme la dépression; des troubles d'anxiété; la douleur; la toxicomanie; des dysfonctionnements sexuels comme un dysfonctionnement érectile; l'épilepsie; le glaucome; des troubles urinaires comme l'incontinence urinaire; les bouffées de chaleur ménopausiques et post-ménopausiques; le diabète de type 2; des troubles de l'alimentation comme les troubles d'alimentation compulsive (binge eating), l'anorexie nerveuse et la boulimie; la prise de poids associée à l'administration de médicaments antipsychotiques, le syndrome de tension prémenstruelle, les troubles du sommeil; et en particulier l'obésité.

17. Composition pharmaceutique selon l'une quelconque des revendications 15 ou 16, où le sujet est un être humain.

18. Composition pharmaceutique selon l'une quelconque des revendications 15 ou 16, où le sujet est un animal.

19. Composition pharmaceutique destinée à moduler la fonction du récepteur 5-HT_{2C}, comprenant la mise en contact du récepteur avec une quantité efficace stimulante ou inhibitrice, de préférence stimulante, d'un composé selon l'une quelconque des revendications 1 à 8.

20. Procédé de préparation d'un composé de formule (Ib) selon l'une quelconque des revendications 7 ou 8, ce procédé comprenant:
a) la réaction d'un composé de formule (II) 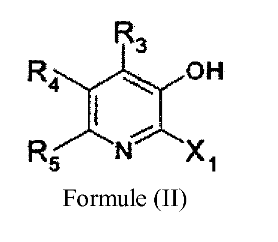 dans laquelle:
X₁ est choisi parmi F, Cl, Br et I,
R₃-R₅ sont chacun indépendamment H, halogène, méthyle ou méthoxy, à condition qu'au moins l'un des R₃-R₅ soit l'hydrogène,
avec un composé de formule (III) 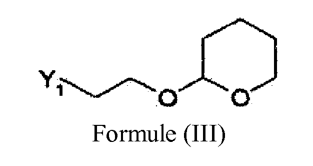 dans laquelle Y₁ est un groupe partant approprié choisi parmi Cl, Br, I, OTs ou OMs; en présence d'une base comme le carbonate de potassium, la triéthylamine ou la pyridine, dans un solvant comme l'acétonitrile, pour l'obtention d'un composé de formule (IV) 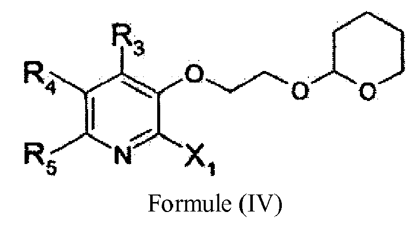 dans laquelle X₁ et R₃-R₅ sont tels que définis ci-dessus;
b) la réaction du composé de formule (IV) avec un composé de formule (Vb) en présence d'une base comme le tert-butylate de potassium, dans un solvant comme le toluène 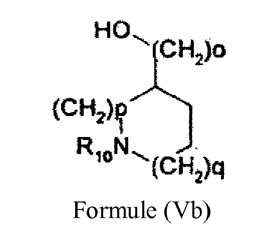 où:
o est un entier de 0 à 2;
p est un entier de 0 à 2, o et p n'étant de préférence pas 0 tous les deux;
q est un entier de 0 à 1 ;
R₁₀ est H ou alkyle en C₁-C₄, de préférence H ou méthyle;
pour l'obtention d'un composé de formule (VIb): 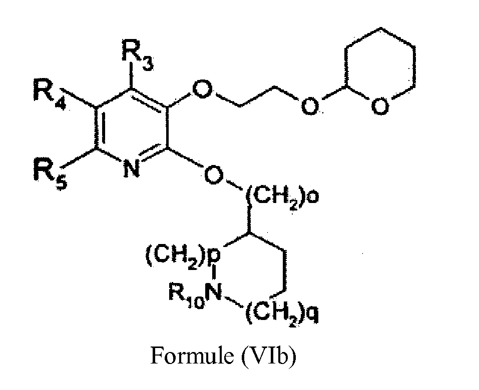 dans laquelle:
R₃-R₅ et R₁₀, o, p et q sont tels que définis ci-dessus,
c) le traitement du composé de formule (VIb) avec un acide aqueux comme l'acide acétique aqueux ou l'acide chlorhydrique aqueux, pour l'obtention d'un composé de formule (VIIb): 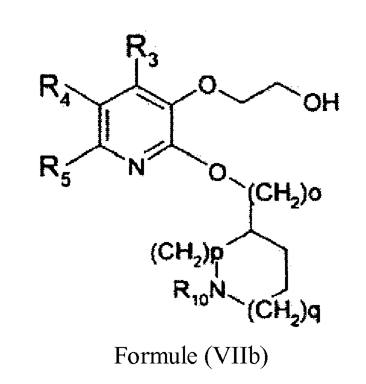 dans laquelle:
R₃-R₅ et R₁₀, o, p et q sont tels que définis ci-dessus,
d) la réaction du composé de formule (VIIb) avec un composé de formule (VIII) en présence d'une base comme le tert-butylate de potassium, dans un solvant comme l'éther de méthyle et tert-butyle ou le toluène 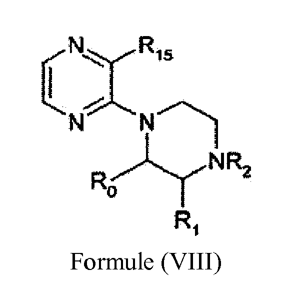 où
R₀ et R₁ sont chacun indépendamment H ou CH₃;
R₂ est choisi parmi (alcoxy en C₁-C₄)carbonyle, benzyle, trityle, alkyle en C₁-C₄, 2- hydroxyéthyle, 2-cyanoéthyle, tétrahydropyran-2-yle et acyle en C₁-C₄,
R₁₅ est un halogène comme le chlore,
pour l'obtention d'un composé de formule (Ib) dans lequel X = N et Y = N: 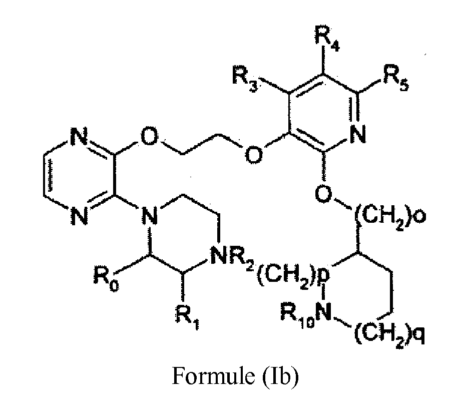 où R₀-R₅, R₁₀, o, p et q sont tels que définis ci-dessus;
e) si désiré, la séparation d'un racémate obtenu en les isomères optiques et/ou la formation d'un sel d'addition d'acide avec un acide organique ou inorganique,
f) si R₂, dans la formule (Ib) après l'étape d), est un groupe protecteur d'azote, comme t-BOC, trityle et benzyle, la séparation dudit groupe protecteur d'azote, comme dans des conditions acides (par exemple avec de l'acide trifluoroacétique dans un solvant comme le chloroforme), des conditions hydrogénolytiques ou non hydrogénolytiques, pour l'obtention du composé de formule (Ib) dans lequel R₂ est l'hydrogène.

21. Procédé de préparation d'un composé de formule (Ib) selon l'une quelconque des revendications 7 ou 8, ce procédé comprenant:
a) la réaction d'un composé de formule (IX) avec un agent de benzylation comme le chlorure de benzyle, le bromure de benzyle ou le tosylate de benzyle, en présence d'une base: 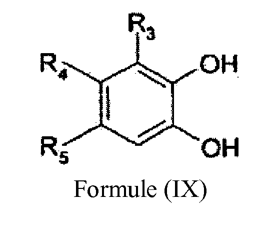 où R₃-R₅ sont chacun indépendamment H, halogène, méthyle ou méthoxy, à condition qu'au moins l'un des R₃-R₅ soit l'hydrogène, pour l'obtention d'un composé de formule (X): 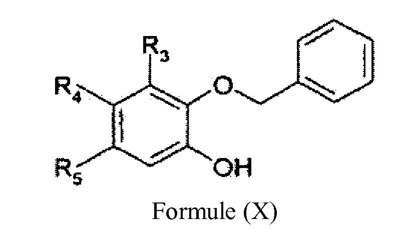 dans laquelle R₃-R₅ sont tels que définis ci-dessus,
b) la réaction du composé de formule (X) avec un composé de formule (XIb) en présence d'une base comme le carbonate de potassium, dans un solvant comme l'acétone: 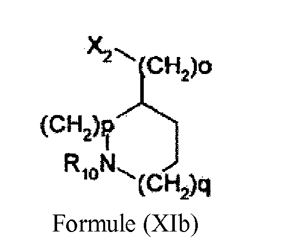 où:
X₂ est halogène, OMs ou OTs;
o est un entier de 0 à 2;
p est un entier de 0 à 2, o et p n'étant de préférence pas 0 tous les deux;
q est un entier de 0 à 1 ;
R₁₀ est H ou alkyle en C₁-C₄, de préférence H ou méthyle;
pour l'obtention d'un composé de formule (XIIb): 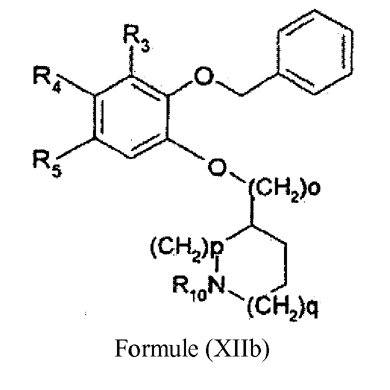 dans laquelle R₃-R₅, R₁₀, o, p et q sont tels que définis ci-dessus,
c) le traitement du composé de formule (XIIb) avec de l'hydrogène en présence d'un catalyseur d'hydrogénation comme le palladium sur du carbone, dans un solvant comme le méthanol, pour l'obtention d'un composé de formule (XIIIb): 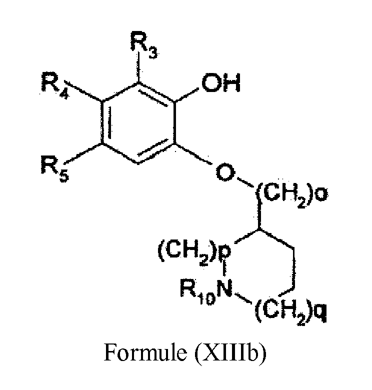 dans laquelle R₃-R₅, R₁₀o, p et q sont tels que définis ci-dessus,
d) la réaction du composé de formule (XIIIb) avec un agent d'hydroxyéthylation comme le carbonate d'éthylène en présence d'une base comme le carbonate de potassium, dans un solvant comme le N,N-diméthylformamide, pour l'obtention d'un composé de formule (XIVb): 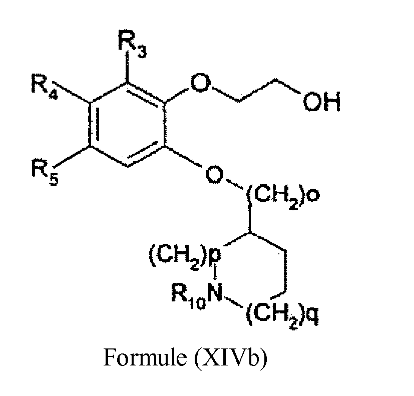 dans laquelle R₃-R₅, R₁₀, o, p et q sont tels que définis ci-dessus;
e) la réaction du composé de formule (XIVa) avec un composé de formule (VIII) en présence d'une base comme le tert-butylate de potassium, dans un solvant comme le N,N-diméthylformamide: 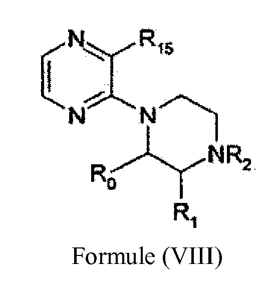 où
R₀ et R₁ sont chacun indépendamment H ou CH₃;
R₂ est choisi parmi (alcoxy en C₁-C₄)carbonyle, benzyle, trityle, alkyle en C₁-C₄, 2- hydroxyéthyle, 2-cyanoéthyle, tétrahydropyran-2-yle et acyle en C₁-C₄,
R₁₅ est un halogène comme le chlore,
pour l'obtention d'un composé de formule (Ib) dans lequel X = N et Y = N: 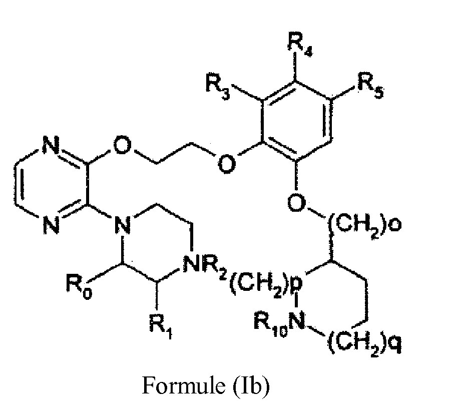 où R₀-R₅, R₁₀, o, p et q sont tels que définis ci-dessus;
f) si désiré, la séparation d'un racémate obtenu en les isomères optiques et/ou la formation d'un sel d'addition d'acide avec un acide organique ou inorganique,
g) si R₂, dans la formule (Ib) après l'étape e), est un groupe protecteur d'azote, comme t-BOC, trityle et benzyle, la séparation dudit groupe protecteur d'azote, comme dans des conditions acides (par exemple avec de l'acide trifluoroacétique dans un solvant comme le chloroforme), des conditions hydrogénolytiques ou non hydrogénolytiques, pour l'obtention du composé de formule (Ib) dans lequel R₂ est l'hydrogène.

22. Procédé de préparation d'un composé de formule (Ib) selon l'une quelconque des revendications 7 ou 8, ce procédé comprenant:
a) la réaction d'un composé de formule (XV) avec un composé choisi parmi le chlorure de benzyle, le bromure de benzyle, l'iodure de benzyle, le tosylate de benzyle et le mésylate de benzyle en présence d'une base comme le carbonate de potassium, dans un solvant comme le N,N-diméthylformamide 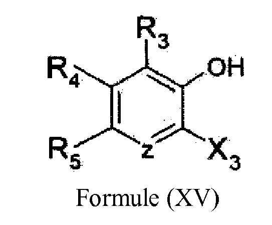 dans laquelle:
X₁ est choisi parmi Cl, Br et I, Z est CH ou N,
R₃-R₅ sont chacun indépendamment H, halogène, méthyle ou méthoxy, à condition qu'au moins l'un des R₃-R₅ soit l'hydrogène, pour l'obtention d'un composé de formule (XVI): 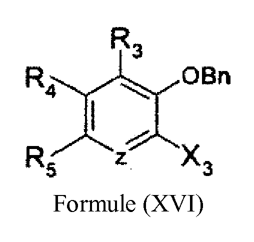
dans laquelle R₃-R₅, X₃ et Z sont tels que définis ci-dessus;
b) la réaction du composé de formule (XVI) avec un composé de formule (Vb) en présence d'une base comme le tert-butylate de sodium, dans un solvant comme le N,N-diméthylformamide, 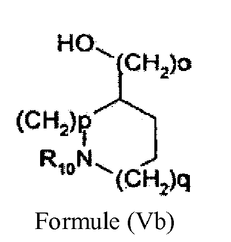 dans laquelle:
o est un entier de 0 à 2;
p est un entier de 0 à 2, o et p n'étant de préférence pas 0 tous les deux;
q est un entier de 0 à 1 ;
R₁₀ est H ou alkyle en C₁-C₄, de préférence H ou méthyle;
pour l'obtention d'un composé de formule (XVIIb): 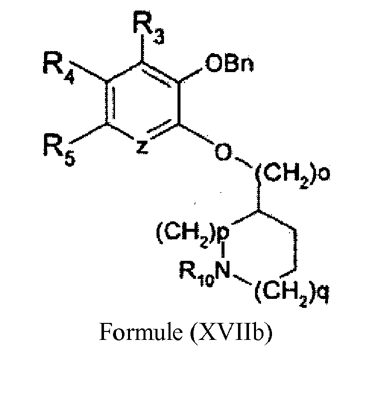 dans laquelle R₃-R₅, et R₁₀, o, p, q et Z sont tels que définis ci-dessus;
c) le traitement du composé de formule (XVIIb) avec un catalyseur d'hydrogénation à l'aide d'une source d'hydrogène appropriée comme le formiate d'ammonium, puis le chauffage en présence d'un agent d'hydroxyéthylation, de préférence le carbonate d'éthylène, et d'une base comme le carbonate de potassium, dans un solvant comme le N,N-diméthylformamide, pour l'obtention d'un composé de formule (VIIb): 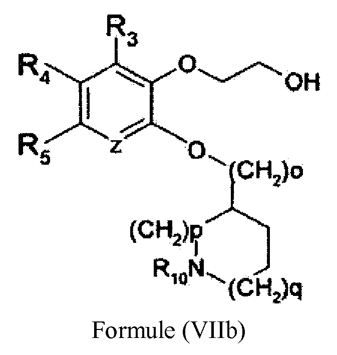 dans laquelle R₃-R₅ et R₁₀, o, p, q et Z sont tels que définis ci-dessus,
d) la réaction du composé de formule (VIIb) avec un composé de formule (VIII) en présence d'une base comme le tert-butylate de sodium, dans un solvant comme le N,N-diméthylformamide: 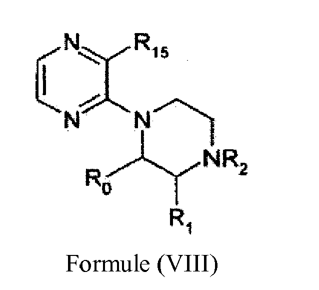 où
R₀ et R₁ sont chacun indépendamment H ou CH₃;
R₂ est choisi parmi (alcoxy en C₁-C₄)carbonyle, benzyle, trityle, alkyle en C₁-C₄, 2- hydroxyéthyle, 2-cyanoéthyle, tétrahydropyran-2-yle et acyle en C₁-C₄,
R₁₅ est un halogène comme le chlore,
pour l'obtention d'un composé de formule (Ib) dans lequel X = N et Y = N: 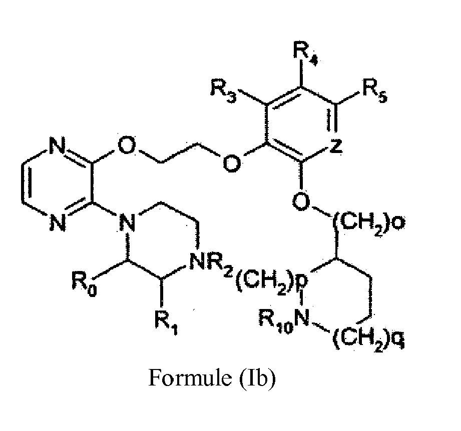 où R₀-R₅, R₁₀, o, p, q et Z sont tels que définis ci-dessus;
e) si désiré, la séparation d'un racémate obtenu en les isomères optiques et/ou la formation d'un sel d'addition d'acide avec un acide organique ou inorganique,
f) si R₂, dans la formule (Ib) après l'étape d), est un groupe protecteur d'azote, comme t-BOC, trityle et benzyle, la séparation dudit groupe protecteur d'azote, comme dans des conditions acides (par exemple avec de l'acide trifluoroacétique dans un solvant comme le chloroforme), des conditions hydrogénolytiques ou non hydrogénolytiques, pour l'obtention du composé de formule (Ib) dans lequel R₂ est l'hydrogène.

23. Composition pharmaceutique pour le traitement d'un trouble ou d'un état, comprenant l'administration à un sujet en ayant besoin d'une quantité efficace d'un composé selon l'une quelconque des revendications 1 à 8, la condition ou l'état étant choisis parmi des troubles de la mémoire comprenant la maladie d'Alzheimer; la schizophrénie; des troubles de l'humeur comme la dépression; des troubles d'anxiété; la douleur; la toxicomanie; des dysfonctionnements sexuels comme un dysfonctionnement érectile; l'épilepsie; le glaucome; des troubles urinaires comme l'incontinence urinaire; les bouffées de chaleur ménopausiques et post-ménopausiques; le diabète de type 2; des troubles de l'alimentation comme les troubles d'alimentation compulsive (binge eating), l'anorexie nerveuse et la boulimie; la prise de poids associée à l'administration de médicaments antipsychotiques, le syndrome de tension prémenstruelle, les troubles du sommeil; et en particulier l'obésité.

24. Utilisation d'un composé selon l'une quelconque des revendications 1 à 8 pour la fabrication d'un médicament destiné au traitement ou à la prévention des troubles de la mémoire comprenant la maladie d'Alzheimer; de la schizophrénie; des troubles de l'humeur comme la dépression; des troubles d'anxiété; de la douleur; de la toxicomanie; des dysfonctionnements sexuels comme un dysfonctionnement érectile; de l'épilepsie; du glaucome; des troubles urinaires comme l'incontinence urinaire; des bouffées de chaleur ménopausiques et post-ménopausiques; du diabète de type 2; des troubles de l'alimentation comme les troubles d'alimentation compulsive (binge eating), l'anorexie nerveuse et la boulimie; de la prise de poids associée à l'administration de médicaments antipsychotiques, du syndrome de tension prémenstruelle, des troubles du sommeil; et en particulier de l'obésité.
